# EUROPEAN PATENT SPECIFICATION

(11) **EP 3 559 278 B1**
(45) Date of publication and mention of the grant of the patent: **14.08.2024**
(21) Application number: 17838104.2
(22) Date of filing: 21.12.2017
(51) Int. Cl.: C12Q 1/6897, G01N 33/50, G01N 33/536, G01N 33/554, G01N 33/569, G01N 33/58

(54) **UNIVERSAL BIOSENSOR SYSTEM FOR ANALYTE DETECTION**
UNIVERSELLES BIOSENSORSYSTEM ZUR DETEKTION VON ANALYTEN
SYSTÈME DE BIOCAPTEUR UNIVERSEL PERMETTANT LA DÉTECTION D'ANALYTE

(30) Priority: 22.12.2016 US 201662438068 P; 06.07.2017 US 201715642800
(43) Date of publication of application: 30.10.2019
(73) Proprietor: Fundamental Solutions Corporation, Easton, PA 18042 (US)
(72) Inventor: ZUPANCIC, Thomas, J., Powell, OH 43065 (US); KITTLE, Joseph, D., The Plains, OH 45780 (US); ZENG, Lingchun, Upper Arlington, OH 43221 (US); VEDAMOORTHY, Srikanth, New Albany, OH 43054 (US); BRODY, Richard, S., Columbus, OH 43214 (US); WILLIAMS, Marvin, R., Easton, PA 18042 (US)
(74) Representative: Carpmaels & Ransford LLP
(86) International application number: PCT/US2017/067787
(87) International publication number: WO 2018/119173

(56) References cited:
- WO-A1-02/068473
- WO-A1-2016/149109
- WO-A2-2016/161088
- US-A1- 2003 064 362
- US-A1- 2013 149 775

## Description

### BACKGROUND OF THE INVENTION

The described invention relates in general to systems, devices, reagents, and methods for detecting various analytes of interest in biological samples or other sample types, and more specifically to a biosensor-based system for detecting and identifying analytes of interest in real-time based on the emission of a detectable signal when the biosensor reacts with an analyte of interest in a sample being tested. The following patents provide additional background information regarding the technology of the present invention: U.S. Patent Nos. 9,023,640; 9,752,199; 9,850,546; 9,850,547; and 9,850,548.

In generic terms, a biosensor is a system or device for the detection of an analyte that combines a sensitive biological component with a physicochemical detector component. The components of a typical biosensor system include a biological element, a transducer or detector element, and associated electronics or signal processors that display test results in a meaningful and useful manner. The biological element typically includes biological material such as tissue, microorganisms, organelles, cell receptors, enzymes, antibodies, nucleic acids, and the like that may be created by known biological engineering processes. The transducer or detector element works in a physicochemical manner (e.g., optical, piezoelectric, and/or electrochemical) that transforms the signal resulting from the interaction of the analyte with the biological element into another signal that can be more easily measured and quantified. Biosensors originated from the integration of molecular biology and information technology (e.g., microcircuits, optical fibers, etc.) to qualify or quantify biomolecule- analyte interactions such as antibody-antigen interactions. Considering that there is great demand for rapid, sensitive, easy-to-handle, and cost effective detection tools for detecting infectious agents, pathogens or/and toxins in food (see, for example, Mead et al., Food Related Illness and Death in the United States, Emerging Infectious Diseases; Vol. 5, No. 5, September-October 1999 (607-625), there is an ongoing need for the utilization of biosensors in real-time, field-portable devices and instruments for the detection and identification of infectious agents, pathogenic microorganisms, toxins, and other contaminants in foods.

WO 02/068473 describes a biosensor for detecting chemical agents. US 2003/064362 describes a biosensor for the detection of toxic substances. US 2013/149775 describes a device for the rapid detection of infectious agents. WO 2016/149109 describes a universal antibody-mediated biosensor. WO 2016/161088 describes a biosensor system for the rapid detection of analytes.

### SUMMARY OF THE INVENTION

The invention is defined in the appended claims. The invention provides a biosensor system for the detection of target analytes, comprising:
(a) a living biological cell of a predetermined type;
(b) a signal-generating reporter within the living biological cell, wherein the signal-generating reporter is responsive to predetermined changes occurring within the living biological cell;
(c) a signal transduction pathway or activator mechanism associated with the signal-generating reporter, wherein the signal transduction pathway or activator mechanism is operative to induce the predetermined changes within the living biological cell;
(d) a universal detector element associated with the activator mechanism, wherein the universal detector element is operative to trigger the activator mechanism, and wherein the universal detector element comprises an antibody variable diversity joining (VDJ) region or Fab fragment;
(e) an analyte binding element associated with the universal detector element, wherein the analyte binding element is specific to both the universal detector element and a target analyte; and
(f) wherein upon the binding of an analyte binding element to which a target analyte is also bound to the universal detector element, the universal detector element triggers the activator mechanism causing the predetermined changes to occur within the living biological cell, thereby causing the signal-generating reporter to generate a detectable signal,
wherein the cell is not the human body at the various stages of its formation and development.
The following provides a summary of certain exemplary embodiments of the present invention. This summary is not an extensive overview and is not intended to identify key or critical aspects or elements of the present invention or to delineate its scope. However, it is to be understood that the use of indefinite articles in the language used to described and claim the present invention is not intended in any way to limit the described system to single components or elements. Rather the use of "a" or "an" herein should be interpreted as meaning "at least one" or "one or more".

Additional features and aspects of the present invention will become apparent to those of ordinary skill in the art upon reading and understanding the following detailed description of the exemplary embodiments. The drawings and associated descriptions are to be regarded as illustrative and not restrictive in nature.

### BRIEF DESCRIPTION OF THE DRAWINGS

The accompanying drawings, which are incorporated into and form a part of the specification, schematically illustrate one or more examples for reference purposes only.
FIGS. 1a-b are illustrations of a first biosensor, wherein Jurkat T cells have been engineered to produce aequorin and to express the transmembrane non-antibody signal transducing element IgGbp-CD3ζ;
FIGS. 2a-b are illustrations of a second biosensor, wherein MC/9 mast cells have been engineered to produce aequorin, and wherein the MC/9 cells express the native receptor FcεRI, which binds to the soluble non-antibody signal transducing element IgGbp-IgE;
FIGS. 3a-b are illustrations of a third biosensor, wherein MC/9 mast cells have been engineered to produce aequorin, and wherein the MC/9 cells express the native receptor FcεRI, which binds to the soluble non-antibody signal transducing element IgGbp-IgE, which has been excreted by the MC/9 mast cells;
FIGS. 4 is an illustration of a fourth biosensor, wherein biosensor cells have been engineered to produce aequorin and to express the transmembrane non-antibody signal transducing element mSA-CD3ζ, which binds to a biotinylated detector element; and
FIGS. 5 is an illustration of a fifth biosensor, wherein biosensor cells have been engineered to produce aequorin and to express the transmembrane non-antibody signal transducing element mSA-CD3ζ, which binds to a biotinylated detector element.

### DETAILED DESCRIPTION OF THE INVENTION

Exemplary embodiments of the present invention are now described. Accordingly, the following embodiments of the invention are set forth without any loss of generality to, and without imposing limitations upon, the claimed invention.

The present invention relates in general to systems for detecting various analytes and/or other targets of interest in biological samples or other sample types, and more specifically to a biosensor-based system for detecting and identifying analytes of interest in real time based on the emission of a detectable signal when the biosensor reacts with an analyte of interest in a sample being tested. The engineered cells of the present invention are extremely sensitive and effective biosensors and because these biosensor cells have an intrinsic detection capacity, they provide a versatile system that can be readily adapted to detect a wide variety of different infectious agents or other targets by simply selecting alternative soluble detector (e.g., antibody) molecules with specificity for a particular pathogen or other target of interest. Furthermore, the system of this invention can be readily configured for multiplex detection of several infectious agents or other analytes in a single assay, providing for great flexibility and utility. The versatility of the present invention is derived from a unique combination of elements and in particular from the combination of a universal biosensor cell with a specific soluble detector (e.g., antibody). The universal biosensor cell has the capacity to respond to the presence of essentially any target molecule that can be recognized by the detector molecule. Because, in some embodiments, the detector or detector antibody is added to the system as a soluble factor, the system may be configured to detect an alternative target by simply selecting an appropriate alternate detector or detector antibody. The specificity of the disclosed system is determined by the detector molecule, which is selected based on its specificity and affinity for a target molecule that is characteristic of an infectious agent or other target analyte. The combination of this universal biosensor cell and soluble detector also enables the construction of multiplex assays by simply including a plurality of detector molecules (e.g., antibodies) within the test system, wherein the target molecules are selected based on their specificity for alternative infectious agents or other analytes.

Genetic manipulation and modification of the biosensor cell types used with this invention typically involve the use of appropriately selected gene delivery vehicles that contain genetic elements that function efficiently in the cell type of choice. For example, it is useful to employ a promoter element that directs high level expression of introduced transgenes in the specific biosensor cell of choice. In an exemplary embodiment of this invention, such a promoter element may be derived directly from the biosensor cell itself and then used to express a transgene of interest. In another embodiment of this invention, an appropriate element may be determined empirically by comparing the function of alternative promoter elements in the context of alternative gene delivery vehicles in order to identify effective promoter, transgene, vector combinations for the cell type of choice. Transgenes such as the gene encoding a luminescent reporter protein may be introduced into the biosensor cell using standard techniques such as electroporation or chemical transfection reagents such as, for example, lipofectamine. Other genetic engineering methods known to those of ordinary skill in the art are also compatible with the present invention.

An exemplary embodiment includes a living, engineered biosensor cell, wherein the living engineered biosensor cell is typically a component of the mammalian immune system; a reporter protein, wherein the reporter protein is expressed by and present within the living, engineered cell, and wherein the reporter protein emits a detectable signal in response to certain predetermined changes in the cytosol of the living, engineered cell; a signal transduction pathway expressed by the living, engineered cell, wherein the signal transduction pathway controls a biological or biochemical process within the cytosol of the living, engineered cell, and wherein the at least one biological or biochemical process, when it occurs, causes the reporter protein to emit a detectable signal; at least one type of detector molecule, wherein each detector molecule is adapted to bind to a specific analyte; at least one analyte, wherein the at least one analyte binds to the detector molecule that is specific to that analyte; and a plurality of transmembrane non-antibody signal transducing elements expressed by the living, engineered cell, wherein each signal transducing element is adapted to receive a detector molecule that is itself adapted to receive an analyte. Upon the binding of a sufficient number of analytes to a sufficient number of detector molecules that are themselves bound to transmembrane non-antibody signal transducing elements, an aggregation of signal transducing elements occurs on the cell surface, the signal transduction pathway is activated, the biological or biochemical process occurs, and the detectable signal is emitted by the reporter protein. This system may also include a device for mixing the living cells together with soluble components and a sample containing an analyte or infectious agent of interest while maintaining the viability and functionality of the living biosensor cell, and a detector for detecting the signal emitted by the biosensor cell.

Another exemplary embodiment includes a living, engineered cell, wherein the living engineered cell is a component of the mammalian immune system, wherein the living engineered cell is a mast cell, and wherein the mast cell expresses at least one predetermined receptor; a reporter protein, wherein the reporter protein is aequorin that is expressed by the living, engineered cell, and wherein the aequorin emits a detectable signal of light in response to certain predetermined changes in the cytosol of the living, engineered cell; a signal transduction pathway expressed by the living, engineered cell, wherein the signal transduction pathway controls a biochemical process within the cytosol of the living, engineered cell, wherein the biochemical process controlled by the signal transduction pathway further includes an increase in intracellular calcium, and wherein the increase in intracellular calcium, when it occurs, causes the aequorin to emit detectable light; at least one type of detector molecule, wherein each detector molecule is adapted to bind to a specific analyte; at least one analyte, wherein the at least one analyte binds to the detector molecule that is specific to that analyte; and a plurality of soluble non-antibody signal transducing elements. Each signal transducing element is adapted to bind to the at least one predetermined receptor and to receive a detector molecule. Upon the binding of a sufficient number of analytes to a sufficient number of detector molecules to a sufficient number of transmembrane non-antibody signal transducing elements that are themselves bound to the at least one type of predetermined receptor, an aggregation of the receptors occurs on the cell surface, the signal transduction pathway is activated, the increase in intracellular calcium occurs, and detectable light is emitted by the aequorin. This system may also include a device for mixing the living cells together with soluble components and a sample containing an analyte or infectious agent of interest while maintaining the viability and functionality of the living biosensor cell, and a detector for detecting the signal emitted by the biosensor cell.

Still another exemplary embodiment includes a biosensor for the rapid detection, wherein the biosensor futher includes a living, engineered cell, wherein the living, engineered cell is derived from a cellular component of the mammalian immune system (i.e., an immunocyte); a reporter protein, wherein the reporter protein is engineered into and expressed by the living, engineered cell, and wherein the reporter protein emits a detectable signal in response to certain predetermined changes in the cytosol of the living, engineered cell; a signal transduction pathway engineered into or occurring naturally within the living, engineered cell, wherein the signal transduction pathway controls a biological process within the cytosol of the living, engineered biosensor cell, and wherein the biological process, when it occurs, causes the reporter protein to emit a detectable signal; and a plurality of non-antibody signal transducing elements that directly or indirectly bind to an analyte in a sample to be analyzed, wherein the bound non-antibody signal transducing elements then cooperate with the biosensor cell to directly or indirectly activate the signal transduction pathway.

### Living Biological Cell

Exemplary embodiments of this invention include a living, engineered biosensor cell that is typically a component of the mammalian immune system, e.g., an immunocyte. In certain embodiments of this invention, the biosensor cell is a human or mouse B cell. B cells or B lymphocytes, are a type of white blood cell of the lymphocyte subtype that function in the humoral immunity component of the adaptive immune system by secreting antibodies. In other embodiments of this invention, the biosensor cell is a human or mouse T cell. T cells or T lymphocytes are another type of lymphocyte that play a central role in cell-mediated immunity as part of the adaptive immune system. T cells are distinguishable from other lymphocytes due to the presence of a T-cell receptor on the cell surface. In other embodiments of this invention, the biosensor cell is a mast cell. A mast cell is also a type of white blood cell known as a granulocyte that is derived from the myeloid stem cell that is a part of the immune and neuroimmune systems. Other types of cells are compatible with this invention, including basophils, which are another type of white blood cell, and which are similar in both appearance and function to mast cells.

In other embodiments of this invention, the living biological cell can be a prokaryotic cell or a eukaryotic cell such as a eukaryotic cell that includes a Ca²⁺ signaling system. The living cell can be a yeast cell or an insect cell such as an insect cell that is a Drosophila Schneider 2 (S2) cell, an sf9 cell, or an insect cell that has been engineered to use aequorin as the reporter. The living biological cell can be a mammalian cell such as an HEK cell, a CHO cell, a COS cell, or a 3T3 cell. The living biological cell can be an engineered cell. The engineered cell can be derived from a native, passaged, or cultured mammalian cell. The engineered cell can be derived from a non-reproducing cell, a drug- or chemically-treated cell, an osmotically-treated cell, a radiated cell, an artificial or synthetic cell, provided that the engineered cell comprises a functional ligand, signal-transduction pathway and reporter. The cell can be an artificial or synthetic cell. The engineered cell can be derived from a plant cell, an animal cell, an insect cell or other non-mammalian animal cell, a component of a mammalian immune system, a follicular dendritic cell, natural killer cell, macrophage, monocyte, mononuclear phagocyte, neutrophil, eosinophil, or basophil. The engineered cell can also be a cell that expresses Fc receptor types, such as B lymphocytes, follicular dendritic cells, natural killer cells, macrophages, neutrophils, eosinophils, basophils and mast cells. In certain embodiments, the cell can be any prokaryotic or eukaryotic cell that has a suitable receptor, signaling pathway, and signal output method, either naturally, through genetic engineering, or through chemical addition. The cell can be an artificial unit provided that it has a functional receptor, signaling pathway, and signal output method. An example of a cell useful in this system is a macrophage cell, such as the human cell line U937, which expresses an Fc receptor on the cell surface. An antigen can be bound to an antibody by addition of the antibody to the target and this antigen-antibody complex will bind to the Fc receptor on the cell and stimulate signaling which results in an increase in intracellular calcium. The cells can be frozen, desiccated, or freeze-dried.

### Signal-Generating Reporter

Exemplary embodiments of this invention include a reporter element, such as a reporter protein or enzyme that is produced or expressed by the living, engineered biosensor cell. The reporter protein emits a detectable signal in response to certain predetermined changes in the cytosol of the living, engineered biosensor cell. In certain embodiments of this invention, the reporter protein is a bioluminescent photoprotein such as aequorin, which is derived from the hydrozoan *Aequorea Victoria.* Aequorin has been previously used for engineering living biosensor cells to produce light signals in response to activation of a wide variety of signal transduction pathways; thus, various methods for manipulating the production of aequorin in living cells are well known to the skilled artisan. In particular, a skilled artisan may select and employ any appropriate gene delivery vehicle such as, for example, bacterial plasmid vectors or viral vectors, for introducing the appropriate genetic material into the biosensor cells. Production of the reporter protein within the biosensor cell will then be controlled by expression of the introduced genetic material. One having ordinary skill in the art will also appreciate that other photoproteins or other types of reporter proteins, enzymes, and molecules may be incorporated into and utilized with various alternate embodiments of the present invention.

In various embodiments of this invention, the reporter can be a protein that has fluorescent properties that undergo a detectable change in response to the activation of the at least one biochemical pathway and a resultant change in the living biological cell. The reporter or reporter protein can be other calcium-sensitive luminescent or fluorescent molecules, such as obelin, thalassicolin, mitrocomin (halistaurin), clytin (phialidin), mnemopsin, berovin, Indo-1, Fura-2, Quin-2, Fluo-3, Rhod-2, calcium green, BAPTA, cameleons (A. Miyawaki et al., (1999) Proc. Natl. Acad. Sci. 96, 213540), or similar molecules. The reporter protein can be a chimeric protein that includes a Ca²⁺ binding domain and an associated fluorescent protein. The associated fluorescent protein can be a green fluorescent protein (GFP). The protein can bind other components of the phosphatidyl inositol pathway (i.e., the pathway used in the embodiments described herein) and change its fluorescence. An example is a fluorescent protein that has been engineered to bind diacylglycerol. The reporter can be an enzyme that is adapted to produce a luminescent or fluorescent signal. The reporter protein can be an enzyme such as luciferase or alkaline phosphatase that yields a luminescent or fluorescent signal respectively. It can also be a fluorescent protein or can include fluorescent, charged, or magnetic nanoparticles, nanodots, or quantum dots. The reporter can be a dye that has fluorescent, ultraviolet, or visible properties, wherein the fluorescent, ultraviolet, or visible properties undergo a detectable change in response to the activation of the at least one biochemical pathway and the resultant change in the living biological cell.

### Activator Mechanism

Exemplary embodiments of this invention include an activator mechanism in the form of a signal transduction pathway expressed by the living, engineered biosensor cell. The signal transduction pathway controls at least one biological process within the cytosol of the living, engineered cell, and the at least one biological process, when it occurs, causes the reporter protein to emit a detectable signal. In certain embodiments of this invention, the signal transduction pathway is any biochemical pathway in which an increase in intracellular Ca2+ concentration is induced in response to activation of a cell surface signal transducing molecule, such as a receptor protein. The biosensor cells used with this invention may be selected from a set of living cells that are capable of producing an increase in cytoplasmic Ca2+ in response to activation of a cell surface signal transduction molecule. For example, B cells, T cells, and mast cells have the capacity to induce an increase in Ca2+ concentration in response to activation of cell surface signal transducing molecules such as the B cell receptor, the T cell receptor, and the Fc epsilon receptor (mast cells), respectively.

Because mammalian cells growing in culture typically generate populations of cells in which specific individual cells may have differing capacities to induce an increase in Ca2+ concentration, it is useful to select for or screen for subpopulations of cells or clonal cell lines that have a robust ability to generate the Ca2+ signal. This may be accomplished by analyzing induction of an aequorin induced flash, for example. In particular, the transfectants created by the introduction of transgenes into a cell are a mixed population of cells derived from a large number of independent gene insertion events. Thus, when constructing a biosensor cell it is useful to screen or select specific subsets of cells or clonal cell lines that have efficient signal transduction capabilities together with useful levels of expression of introduced transgenes. It is particularly useful to use fluorescence-activated cell sorting (FACS) technology to select for subpopulations of high expressing cells or to generate clonal cell lines for this purpose.

As previously stated, aequorin has been used previously for engineering living biosensor cells to produce light signals in response to activation of a wide variety of signal transduction pathways, particularly wherein such signal transduction pathways lead to an increase in cytoplasmic Ca2+ ions within a living cell. In certain embodiments of this invention, biosensor cells that produce aequorin as the reporter protein are charged with coelenterazine (CTZ) prior to their use in a detection assay. This charging step covalently links the aequorin to a hydrophobic prosthetic group (e.g., CTZ) and upon calcium (Ca2+) binding, the CTZ undergoes an irreversible reaction that includes a conformation change, and emits blue light (at 469 nm).

As described above, the signal transduction pathway can transmit a first signal by way of release of calcium ions from the endoplasmic reticulum into the cytosol and a second signal may be released by the reporter in response to the calcium ions. This signaling pathway is the second-messenger cascade found in B cells, T cells, mast cells, macrophages, and other immune cells, wherein crosslinking of the cell surface receptors activates a tyrosine kinase, which then phosphorylates phospholipase C, which then cleaves phosphatidylinositol 4,5-bisphosphate (PIP2) into inositol 1,4,5-trisphosphate (IP3) and diacylglycerol; IP3 then opens calcium channels to release calcium from intracellular stores such as the endoplasmic reticulum or to let in extracellular calcium, thereby elevating the calcium concentration in the cell's cytosol. Depending on the receptor type, cell type, and desired signaling method, alternative second-messenger cascades could be employed, such as a G-protein-adenylyl cyclic-cAMP-protein kinase A cascade. The signal transduction pathway can also transmit a signal by way of release of diacylglycerol, ceramide, or other lipophilic second messenger molecule, wherein the reporter emits a second signal in response to release of diacylglycerol, ceramide, or other lipophilic second messenger molecule. The signal transduction pathway can also transmit a signal by way of release or production of nitric oxide ("NO"), cAMP, cGMP, or other cyclic nucleotide, wherein the reporter emits the second signal in response to this release or production. The signal transduction pathway can also transmit a signal by way of release or production of superoxide, hydrogen peroxide, carbon monoxide, hydrogen sulfide, or other secondary redox messenger, wherein the reporter emits the second signal in response to release or production of the superoxide, hydrogen peroxide, carbon monoxide, hydrogen sulfide, or other secondary redox messenger molecule. In certain embodiments, the activator mechansim includes a change in cell pH or temperature or a change in cell electrical or magnetic properties.

### Universal Detector Element

Exemplary embodiments include various non-antibody signal transducing elements that function as universal detector elements for recogrnizing target analytes. Each signal transducing element is typically adapted to receive, i.e., bind, an analyte binding element (also referred to herein as a "detector molecule") that is itself adapted to receive, i.e., bind, a specific analyte of interest. In one embodiment, the signal transducing element is a transmembrane chimeric fusion protein that is engineered into and expressed on the surface of the biosensor cell, and that is adapted to activate the signal transduction pathway that ultimately results in the reporter protein emitting a detectable signal. In another embodiment, the signal transducing element is a soluble chimeric fusion protein that is adapted to bind to a cell surface signal transducer, such as a native receptor or receptor protein that is adapted to activate the signal transduction pathway that ultimately results in the reporter protein emitting a detectable signal. In still another embodiment, the signal transducing element is a soluble chimeric fusion protein that is engineered into and expressed by the biosensor cell. The soluble chimeric fusion protein is then secreted/excreted into the extracellular space where it binds to a cell surface signal transducer, such as a native receptor or receptor protein that is adapted to activate the signal transduction pathway that ultimately results in the reporter protein emitting a detectable signal.

The chimeric fusion proteins may include: (i) a component of a protein that is adapted to bind to the at least one type of detector molecule (e.g., a soluble antibody); and (ii) a component of a receptor complex normally expressed by the living, engineered biosensor cell. In some embodiments, the component of the protein that is adapted to bind to the at least one type of detector molecule may be derived from a bacterial binding protein (i.e., an antibody binding protein derived from a bacteria) such as, for example, the IgG binding domain of a strep G protein (referred to herein as IgGbp or Igbp in the Figures). Tandem repeats of this IgG binding domain may be included to increase the affinity of the binding protein for the soluble antibody. In an alternate embodiment, the component of the chimeric fusion protein that is adapted to bind to the at least one type of detector molecule is an antibody binding domain derived from a receptor protein such as, for example, the murine Fc gamma RI (FcyRI) receptor. In various exemplary embodiments, the component of the receptor complex normally expressed by the living, engineered biosensor cell is IgM (for B cell biosensors); Igα/β (for B cell biosensors); IgE (for mast cell biosensors); CD19 (for B cell biosensors), CD3zeta (for T cell biosensors), or FcεRI (for mast cell biosensors).

The non-antibody signal transducing elements may include either complete protein sequences or engineered protein fragments such as selected protein domains derived from larger protein molecules. One of ordinary skill in the art will appreciate that fragments of larger molecules may be created using standard genetic engineering techniques such as synthetic gene technology. When fragments of larger proteins are used to engineer antibody binding motifs as aspects of chimeric fusion proteins it is important to design the engineered proteins to ensure proper conformational folding of the selected protein fragments. Therefore, it is useful to include (in the fusion proteins) short spacer or linker elements that do not readily form protein secondary structures. Short combinations of amino acids such as glycine, serine and alanine, for example, may be used for these spacer or linker elements. In an exemplary embodiment, the amino acid sequence glycine (G), serine (S), alanine (A), serine (S), glycine (G), serine (S), glycine (G) is used to separate a binding domain from a component of a receptor complex in an engineered protein molecule (see SEQ ID NO: 19). With regard to the peptide linker or spacer used to connect the detector element to the signal-transducing element or interconnect different segments of a signal-transducing element: the linker typically joins the carboxyl terminus of one element to the amino terminus of another. Peptide linkers may vary from 0 to 25 amino acids in length or any intermediate integer value and typically, but not always, comprise hydrophilic amino acids such as glycine (G) and serine (S).

As previously stated, each signal transducing element binds to a detector molecule that binds to a specific analyte of interest. A detector molecule that has bound to an analyte will either (i) bind to a transmembrane signal transducing element; or (ii) to a signal transducing element that will itself bind to a cell surface signal transducer (e.g., native receptor). In the first situation, upon the binding of a sufficient number of analytes to a sufficient number of detector molecules that are themselves bound to transmembrane non-antibody signal transducing elements, an aggregation of signal transducing elements occurs on the biosensor cell surface, the signal transduction pathway is activated, the biological process occurs, and the detectable signal is emitted by the reporter protein. In the second case, upon the binding of a sufficient number of analytes to a sufficient number of detector molecules to a sufficient number of non-antibody signal transducing elements that are themselves bound to the appropriate native receptor, an aggregation of the receptors occurs on the cell surface, the signal transduction pathway is activated, the increase in intracellular calcium occurs, and detectable light is emitted by the reporter protein.

A first non-antibody signal transducing element includes a bacterial binding protein (IgGbp) fused to the IgM heavy chain constant domain (B cell) with a GSASGSG linker. SEQ ID NO: 1 provides the DNA sequence for signal transducing element IgGbp-IgM and SEQ ID NO: 2 provides the protein sequence for signal transducing element IgGbp-IgM.

A second non-antibody signal transducing element includes a bacterial binding protein (IgGbp) fused to the Igα/β component of the B cell receptor with a GSASGSG linker. SEQ ID NO: 3 provides the DNA sequence for signal transducing element IgGbp-Igα/β and SEQ ID NO: 4 provides the protein sequence for signal transducing element IgGbp-Igα/β.

A third non-antibody signal transducing element includes a bacterial binding protein (IgGbp) fused to the CD3 zeta chain of the T-cell receptor with a GSASGSG linker. SEQ ID NO: 5 provides the DNA sequence for signal transducing element IgGbp-CD3ζ and SEQ ID NO: 6 provides the protein sequence for signal transducing element IgGbp-CD3ζ.

A fourth non-antibody signal transducing element includes the FcyRI antibody binding domain fused to the IgM heavy chain constant domain (B cell) with a GSASGSG linker. SEQ ID NO: 7 provides the DNA sequence for signal transducing element FcyRI-IgM and SEQ ID NO: 8 provides the protein sequence for signal transducing element FcyRI-IgM.

A fifth non-antibody signal transducing element includes the FcyRI antibody binding domain fused to the Igα/β component of the B-cell receptor with a GSASGSG linker. SEQ ID NO: 9 provides the DNA sequence for signal transducing element FcyRI-Igα/β and SEQ ID NO: 10 provides the protein sequence for signal transducing element FcγRI-Igα/β.

A sixth non-antibody signal transducing element includes the FcyRI antibody binding domain fused to the CD3 zeta chain of the T-cell receptor with a GSASGSG linker. SEQ ID NO: 11 provides the DNA sequence for signal transducing element FcγRI-CD3ζ and SEQ ID NO: 12 provides the protein sequence for signal transducing element FcγRI-CD3ζ.

A seventh exemplary non-antibody signal transducing element includes a bacterial binding protein (IgGbp) fused to the IgE constant domain (B cell) with a GSASGSG linker. SEQ ID NO: 13 provides the DNA sequence for signal transducing element IgGbp-IgE and SEQ ID NO: 14 provides the protein sequence for signal transducing element IgGbp-IgE.

An eighth exemplary non-antibody signal transducing element includes the FcyRI antibody binding domain fused to the IgE constant domain (B cell) with a GSASGSG linker. SEQ ID NO: 15 provides the DNA sequence for signal transducing element FcyRI-IgE and SEQ ID NO: 16 provides the protein sequence for signal transducing element FcyRI-IgE.

A ninth exemplary non-antibody signal transducing element includes monomeric streptavidin fused to the CD3 zeta chain of the T-cell receptor with a GSASGSG linker. SEQ ID NO: 17 provides the DNA sequence for signal transducing element mSA-CD3ζ and SEQ ID NO: 18 provides the protein sequence for signal transducing element mSA-CD3ζ. Monomeric streptavidin is a recombinant form of streptavidin that includes mutations that break the streptavidin tetramer into a monomer and to enhance the solubility of the resultant isolated subunit.

In the invention, the universal detector element includes an antibody VDJ region or Fab fragment. The universal detector element can include a T cell VJ region, VDJ region, or other T cell receptor determinant. The universal detector element can include a synthetic peptide; a small organic determinant, which is not a peptide; a protein or peptide determinant; a lectin determinant, a carbohydrate-binding module, or other carbohydrate-binding determinant; a lipid-binding determinant; or a metallothione determinant that binds a metal or other metal-binding determinant.

The universal detector element, can be covalently bound to a signal transduction pathway expressed by the living biological cell. An example is a membrane-anchored antibody where the anchored portion is part of the signal transduction pathway, i.e., transmits a signal from outside the cell into the cell. In some embodiments, the universal detector element is not modular, but is an integral part of the signal transduction pathway, e.g., part of a chimeric protein forming that pathway. The universal detector element can be non-covalently bound to the signal transduction pathway. An example is an antibody externally bound to an Fc receptor on a signal transduction molecule where the molecule bearing the Fc receptor transmits the signal through the membrane. In other embodiments, the universal detector element is modular and the cell containing the signal transduction pathway can be loaded with a universal detector element of choice. The universal detector element can include a determinant that non-covalently binds to a portion of the signal transduction pathway or the universal detector element can include an Fc determinant that non-covalently binds it to an Fc binding portion of the signal transduction pathway. The universal detector element can include a biotin or (strep)avidin determinant that non-covalently binds it to a biotin- or (strep)avidin-binding portion of the signal transduction pathway.

### Analyte Binding Element /Detector Molecule

The invention includes at least one type of analyte binding element, also referered to herein as a "detector molecule", wherein each analyte binding element is adapted to bind to a specific target analyte. The analyte binding element may a soluble antibody that is not in any way expressed by the biosensor cells. The particular analyte binding element used with the present invention is selected based on its ability to unambiguously identify the target analyte of interest. In an exemplary embodiment, the analyte binding element is a soluble antibody such as a commercially available IgG that is specific for a particular analyte, such as an infectious agent. In another exemplary embodiment, the analyte binding element is a biotinylated molecule (or streptavidin-based molecule) that is specific for a predetermined analyte such as, for example, a biotinylated autoantigen molecule that is specific for an anti-autoantigen antibody. A detector or target molecule according to this invention may include an autoantigen or an autoantibody associated with an autoimmune disease. Representative autoimmune diseases or disorders include rheumatoid arthritis (RA), juvenile RA (JRA), diabetes mellitus type 1, systemic lupus erythematosus, Hashimoto's thyroiditis, Graves' disease, scleroderma, celiac disease, Crohn's disease, ulcerative colitis, Sjogren's syndrome, multiple sclerosis, Goodpasture's syndrome, Addison's disease, Wegener's granulomatosis, primary biliary cirrhosis, sclerosing cholangitis, autoimmune hepatitis, polymyalgia rheumatica, temporal arteritis/giant cell arteritis, and Guillain-Barre syndrome. Detector or target molecules may also comprise tumor-specific or tumor-associated antigens or antibodies to such antigens; or biologically active molecules, such as EGF, peptide hormones, including insulin and growth hormone, cytokines, interleukins, interferons, TNF, *etc.* or antibodies to such biologically active molecules.

The system of this invention can further include an analyte binding element that includes an IgG fragment and the IgG fragment can be a single chain antibody or a single chain diabody. The detector can also be an affibody (i.e., engineered binding protein), an aptamer (e.g., DNA or RNA molecule that has been engineered to bind ligands), or a soluble receptor such as a soluble receptor for an infectious virus.

### Target Analytes and Test Sample

An intended use of the present invention is the detection of various analytes that are or might be present within samples to be tested. In an exemplary embodiment of this invention, an analyte that is to be detected will bind to a detector molecule, such as a soluble antibody, that is specific to that analyte. A sample to be tested may be taken from a large number of food sources, including: (i) meats such as beef, pork, lamb, bison, poultry, and seafood; and (ii) plants and vegetables. A sample to be tested may also be taken from many other sources such as water, consumable fluids, preservative fluids, and bodily fluids such as blood. Analytes that may be detected include virtually anything that will bind with specificity to the detector or detector molecule such as chemicals, toxins, and infectious agents such as viruses, bacteria, and other biological materials or agents. In an exemplary embodiment of this invention, the specific infectious agent is *Escherichia coli*, although other infectious agents (such as Salmonella, Listeria, and Campylobacter) and contaminants may be detected with the present invention. *Escherichia coli O157 H7, 026, 045, 0103, O111, O121,* and *O145*, in either separate assays or multiplexed assays, may all potentially be detected using this invention.

The present invention is capable of detecting many different analytes including meat pathogens, and those found on spinach, lettuce, and other vegetables and foods. An analyte may contain one or more epitopes of an antigen or allergen, including both linear or conformation epitopes; it may also contain one or more ligands or receptors recognized by reciprocal receptors or ligands. Exemplary analytes include a bacterium, such as Bacillus (e.g., B. anthracis), Enterobacteriaceae (e.g., Salmonella, Escherichia coli, Yersinia pestis, Klebsiella, and Shigella), Yersinia (e.g., Y. pestis or Y. enterocolitica), Staphylococcus (e.g., S. aureus), Streptococcus, Gonorrheae, Enterococcus (e.g., E. faecalis), Listeria (e.g., L. monocytogenes), Brucella (e.g., B. abortus, B. melitensis, or B. suis), Vibrio (e.g., V. cholerae), Corynebacterium diphtheria, Pseudomonas (e.g., P. pseudomallei or P. aeruginosa), Burkholderia (e.g., B. mallei or B. pseudomallei), Shigella (e.g., S. dysenteriae), Rickettsia (e.g., R. rickettsii, R. prowazekii, or R. typhi), Francisella tularensis, Chlamydia psittaci, Coxiella burnetii, Mycoplasma (e.g., M. mycoides), etc.; allergens, such as peanut dust, mycotoxins, mold spores, or bacterial spores such as Clostridium botulinum and C. perfringens; toxins, such as ricin, mycotoxin, tetrodotoxin, anthrax toxin, botulinum toxin, staphylococcal entertoxin B, or saxitoxin; a virus, such as Adenoviridae (e.g., adenovirus), Arenaviridae (e.g., Machupo virus), Bunyaviridae (e.g., Hantavirus or Rift Valley fever virus), Coronaviridae, Orthomyxoviridae (e.g., influenza viruses), Filoviridae (e.g., Ebola virus and Marburg virus), Flaviviridae (e.g., Japanese encephalitis virus and Yellow fever virus), Hepadnaviridae (e.g., hepatitis B virus), Herpesviridae (e.g., herpes simplex viruses), Papovaviridae (e.g., papilloma viruses), Paramyxoviridae (e.g., respiratory syncytial virus, measles virus, mumps virus, or parainfluenza virus), Parvoviridae, Picornaviridae (e.g., polioviruses), Poxviridae (e.g., variola viruses), Reoviridae (e.g., rotaviruses), Retroviridae (e.g., human T cell lymphotropic viruses (HTLV) and human immunodeficiency viruses (HIV)), Rhabdoviridae (e.g., rabies virus), and Togaviridae (e.g., encephalitis viruses, yellow fever virus, and rubella virus)); a protozoon, such as Cryptosporidium parvum, Encephalitozoa, Plasmodium, Toxoplasma gondii, Acanthamoeba, Entamoeba histolytica, Giardia lamblia, Trichomonas vaginalis, Leishmania, or Trypanosoma (e.g., T. brucei and T. Cruzi); a helminth, such as cestodes (tapeworms), trematodes (flukes), or nematodes (roundworms, e.g., Ascaris lumbricoides, Trichuris trichiura, Necator americanus, or Ancylostoma duodenale); a parasite (e.g., any protozoa or helminths described herein); a fungus, such as Aspergilli, Candidae, Coccidioides immitis, and Cryptococci; an environmental contaminant; a water additive; an agricultural marker; a nucleic acid (e.g., oligonucleotides, polynucleotides, nucleotides, nucleosides, molecules of DNA, or molecules of RNA, including a chromosome, a plasmid, a viral genome, a primer, or a gene); a protein (e.g., a glycoprotein, a metalloprotein, an enzyme, a prion, or an immunoglobulin); a metabolite; a sugar; a lipid; a lipopolysaccharide; a salt; or an ion. Targets also include food-borne pathogens, such as Salmonella (e.g., Salmonella Typhimurium), pathogenic E. coli (e.g., O157:H7), Bacillus (e.g., B. cereus), Clostridium botulinum, Listeria monocytogenes, Yersinia (e.g., Y. enterocolitica), Norovirus (e.g., Norwalk virus), Shigella, Staphylococcus aureus, Toxoplasma gondii, Vibrio (e.g., V. vulnificus, V. cholera, V. parahaemolyticus), Campylobacter jejuni, and Clostridium perfringens; and weaponized pathogens, such as Bacillus anthracis, Yersinia pestis, Francisella tularensis, Brucella (e.g., B. suis), Burkholderia mallei, Burkholderia pseudomallei, Shigella, Clostridium botulinum, Variola (e.g., V. major), Filoviridae (e.g., Ebola virus and Marburg virus), Arenaviridae (e.g., Lassa virus and Machupo virus), Clostridium perfringens, any food-borne pathogen (e.g., Salmonella species, Escherichia coli O157:H7, or Shigella), Chlamydia psittaci, Coxiella burnetii, Staphylococcal aureus, Rickettsia (e.g., R. prowazekii or R. rickettsii), Alphavirus (e.g., Venezuelan equine encephalitis virus, eastern equine encephalitis virus, or western equine encephalitis virus), Vibrio cholerae, Cryptosporidium parvum, Henipavirus (e.g., Nipah virus), Bunyaviridae (e.g., Hantavirus or Rift Valley fever virus), Flaviviridae (e.g., Japanese encephalitis virus and Yellow fever virus), and Coccidioides spp.

Epitopes that can be detected as analytes or portions of an analyte are typically antigenic determinant sites on an antigen to which an immunogolublin (or antigen binding fragment thereof) can specifically bind. Epitopes can be formed both from contiguous amino acids or noncontiguous amino acids juxtaposed by tertiary folding of a protein. Epitopes can be found on the Fab (variable) region of immunoglobulins (referred to as "idiotypic determinants") and comprise the immunoglobulin's "idiotype". The epitope and antigen can be naturally occurring or artificially produced. Depending on the nature of the epitope or antigen, the epitope or antigen can be isolated or purified from a matrix or substance of origin, synthesized, or recombinantly produced, for example. Epitopes and antigens useful as analytes can be from a human or non-human animal, plant, bacteria, protozoan, parasite, virus, etc. In some embodiments, the analyte is a polypeptide, nucleic acid molecule, carbohydrate, glycoprotein, lipid, lipoprotein, glycolipid, or small molecule. In some embodiments, the analyte is selected from among a cancer antigen, autoantigen, allergen, endogenous antigen, infectious agent antigen, drug (small molecule) antigen, toxin, venom, biologic antigen, environmental antigen, transplant antigen, and implant antigen.

An analyte may comprise an epitope of a cancer antigen. In some embodiments, the analyte is a tumor-associated antigen. In some embodiments, the analyte is a tumor-specific antigen. In some embodiments of the invention, the analyte is a tumor-associated antigen (TAA), and the TAA is a carbohydrate antigen having one or more post-translational modifications that differ from the wild-type protein, comprises a fusion region of a protein resulting from a gene fusion that is present in malignant cells but not present in non-malignant cells, and/or wherein the TAA comprises a receptor tyrosine kinase (RTK) that is deregulated and/or dysfunctional in tumor cells due to autocrine activation, chromosomal translocations, RTK overexpression, or gain-of-function mutations in the RTK gene or protein. In some embodiments of the invention, the analyte is an immunoglobulin expressed by a B-cell malignancy. Examples of B-cell malignancies include, but are not limited to, non-Hodgkin's lymphoma, Hodgkin's lymphoma, chronic lymphocytic leukemia, mantle cell lymphoma and multiple myeloma. Additional B-cell malignancies include, for example. B-cell prolymphocytic leukemia, lymphoplasmocytic leukemia, splenic marginal zone lymphoma, marginal zone lymphoma (extra-nodal and nodal), plasma cell neoplasms (e.g., plasma cell myeloma, plasmacytoma, monoclonal immunoglobulin deposition diseases, heavy chain diseases), and follicular lymphoma (e.g., Grades I, II, III, or IV).

In some embodiments, the analyte is a tumor-associated antigen derived from tumor cells obtained from the subject. In some embodiments, the tumor-associated antigen is one or more antigens selected from among 17-1A, 707-AP, AFP, Annexin II, ART-4, BAGE, BAGE-1, .beta.-catenin, BCG, bcr/abl, Bcr/abl e14a2 fusion junction, bcr-abl (b3a2), bcr-abl (b3a2), bcr-abl p190 (ela2), bcr-abl p210 (b2a2), bcr-abl p210 (b3a2), bcr-abl p210 (b3a2), bullous pemphigoid antigen-1, CA19-9, CA125, CA215, CAG-3, CAMEL, Cancer-testis antigen, Caspase-8, CCL3, CCL4, CD16, CD20, CD3, CD30, CD55, CD63, CDC27, CDK-4, CDR3, CEA, cluster 5, cluster-5A, cyclin-dependent kinase-4, Cyp-B, DAM-10, DAM-6, Dekcain, E7, EGFR, EGFRvIII, EGP40, ELF2 M, EpCAM, FucGM1, G250, GA733, GAGE, GAGE-1-8, gastrin cancer associated antigen, GD2, GD3, globoH, glycophorin, GM1, GM2, GM3, GnTV, Gn-T-V, gp100, Her-2/neu, HERV-K-ME, high molecular weight-associated antigen, high molecular weight proteo-glycan (HMPG), HPV-16 E6, HPV-16 E7, HPVE6, HSP70-2M, HST-2, hTERT, human chorionic gonadotropin (HCG), Human milk fat globule (HMFG), iCE, KIAA0205, KK-LC-1, KM-HN-1, L6, LAGE-1, Lcose4Cer, LDLR/FUT, Lewis A, Lewis v/b, M protein, MAGE-1, MVC, MAGE-A1-12, MAGE-C2, MAHGE-3, MART-1/Melan-A, MC1R, ME491, MUC1, MUC2, mucin, MUM-1, MUM-2, MUM-3, mutated p53, Myosin, MZ2-E, N9 neuraminidase, NA88, NA88-A, nasopharyngeal carcinoma antigen, NGA, NK1/c-3, Novel bcr/ablk fusion BCR exons 1, 13, 14 with ABL exons 4, NY-ESO-1/LAGE-2, NY-ESO-1b, OC125, osteosarcoma associated antigen-1, P15, p190 mimor bcr-abl (ela2), p53, Pm1/RARa, Polysialic acid, PRAME, PSA, PSM, RU1, RU2, SAGE, SART-1, SART-2, SART-3, Sialyl LeA, Sp17, SSX-2, SSX-4, surface immunoglobulin, TAG-1, TAG-2, TEL/AML1, TPI, TRAG-3, TRP-1 (gp75), TRP-2, TRP2-INT2, hTRT, tumor associated glycoprotein-72 (TAG-72), tyrosinase, u-PA, WT1, and XAGE-1b, or an immunogenic fragment of any of the foregoing antigens. In some embodiments, the tumor associated antigen is identified by the SEREX (serological analysis of recombinant cDNA expression library) approach or based on the serological screening of cDNA expression library generated from tumor tissues of various origin or cancer cell lines, and identifying immunogenic tumor proteins based on their reactivity with autologous patient sera. In some embodiments, the analyte is a tumor-associated antigen that is acarbohydrate antigen having one or more post-translational modifications that differ from the wild-type protein. In some embodiments, the tumor-associated antigen comprises a fusion region of a protein resulting from a gene fusion that is resent in malignant cells but not present in non-malignant cells. In some embodiments, the tumor-associated antigen comprises a receptor tyrosine kinase that is deregulated and/or dysfunctional in tumor cells due to autocrine activation, chromosomal translocations, RTK overexpression, or gain-of-function mutations in the RTK gene or protein.

The analyte may comprise an epitope of an antigen of an infectious or noninfectious agent that can be either pathogenic or non-pathogenic to the subject. The analyte can be derived from a mutualistic, parasitic, or commensal microorganism, including any microorganism in a animal or plant biome, such as probiotic or commensal microorganisms in the human digestive tract, mucosal surfaces, or epithelium. In some embodiments, the bacterial pathogen is selected from among Acinetobacter baumannii (formerly Acinetobacter calcoaceticus), Actinobacillus, Actinomyces pyogenes (formerly Corynebacterium pyogenes), Actinomyces israelii, nocardia asteroids, N. brasiliensis, Aeromonas hydrophila, Amycolata autotrophica, Archanobacterium haemolyticum (formerly Corynebacterium haemolyticum), Arizona hinshawii--all serotypes, Bacillus anthracis, Bacteroides fragilis, Bartonella henselae, B. quintana, B. vinsonii, Bordetella including B. pertussis, Borrelia recurrentis, B. burgdorferi, Burkholderia (formerly Pseudomonas species) except those listed in BSL III), Campylobacter coli, C. fetus, C. jejuni, Chlamydia psittaci, C. trachomatis, C. pneumonia, Clostridium botulinum (neurotoxin producing species), Clostridium botulinum neurotoxins, Cl. chauvoei, Cl. haemolyticum, Cl. histolyticum, Cl. novyi, Cl. septicum, Cl. Tetani, Cl. Perfirngens epsilon toxin, Corynebacterium diphtheriae, C. pseudotuberculosis, C. renale, Dermatophilus congolensis, Edwardsiella tarda, Erysipelothrix rhusiopathiae, Escherichia coli--all enteropathogenic, enterotoxigenic, enteroinvasive and strains bearing K1 antigen, including E. coli O157:H7, Haemophilus ducreyi, H. influenzae, Helicobacter pylori, Klebsiella--all species except K. oxytoca (RG1), Legionella including L. pneumophila, Leptospira interrogans--all serotypes, Listeria, Moraxella, Mycobacterium (except those listed in BSL III) including M. avium complex, M. asiaticum, M. bovis BCG vaccine strain, M. chelonei, M. fortuitum, M. kansasii, M. leprae, M. malmoense, M. marinum, M. paratuberculosis, M. scrofulaceum, M. simiae, M. szulgai, M. ulcerans, M. xenopi, Mycoplasma, Neisseria gonorrhoeae, N. meningitides, Nocardia asteroides, N. brasiliensis, N. otitidiscaviarum, N. transvalensis, Proteus mirabilis, P. vulgaris, Rhodococcus equi, Salmonella including S. arizonae, S. cholerasuis, S. enteritidis, S. gallinarum-pullorum, S. meleagridis, S. paratyphi, A, B, C, S. typhi, S. typhimurium, Shigella including S. boydii, S. dysenteriae, type 1, S. flexneri, S. sonnei, Sphaerophorus necrophorus, Staphylococcus aureus, Streptobacillus moniliformis, Streptococcus including S. pneumoniae, S. pyogenes, Treponema pallidum, T. carateum, Vibrio cholerae, V. parahemolyticus, V. vulnificus, Yersinia enterocolitica, Bartonella, Brucella including B. abortus, B. canis, B. suis, B. melitensis, Burkholderia (Pseudomonas) mallei, B. pseudomallei, Coxiella burnetii, Francisella tularensis, Mycobacterium bovis (except BCG strain, BSL II-Bacterial Agents Including Chlamydia), M. tuberculosis, Mycobacteria other than tuberculosis (MOTT), Pasteurella multocida type B--"buffalo" and other virulent strains. Rickettsia akari, R. australis, R. canada, R. conorii, R. prowazekii, R. rickettsii, R, siberica, R. tsutsugamushi, R. typhi (R. mooseri), Yersinia pestis.

The analyte can be derived from a viral pathogen. For example, in some embodiments, the analyte is derived from a viral pathogen selected from among Adenoviruses, human--all types, Alphaviruses (Togaviruses), Eastern equine encephalitis virus, Eastern equine encephalomyelitis virus, Venezuelan equine encephalomyelitis vaccine strain TC-83, Western equine encephalomyelitis virus, Arenaviruses, Lymphocytic choriomeningitis virus (non-neurotropic strains), Tacaribe virus complex, Bunyaviruses, Bunyamwera virus, Rift Valley fever virus vaccine strain MP-12, Calciviruses, Coronaviruses. Flaviviruses (Togaviruses)-Group B Arboviruses, Dengue virus serotypes 1, 2, 3, and 4, Yellow fever virus vaccine strain 17D, Hepatitis A, B, C, D, and E viruses, the Cytomegalovirus, Epstein Barr virus, Herpes simplex types 1 and 2, Herpes zoster, Human herpesvirus types 6 and 7, Influenza viruses types A, B, and C, Papovaviruses, Papilloma viruses, Newcastle disease virus, Measles virus, Mumps virus, Parainfluenza viruses types 1, 2, 3, and 4, polyomaviruses (JC virus, BK virus), Respiratory syncytial virus, Human parvovirus (B 19), Coxsackie viruses types A and B, Echoviruses, Polioviruses, Rhinoviruses, Alastrim (Variola minor virus), Smallpox (Variola major virus), Whitepox Reoviruses, Coltivirus, human Rotavirus, and Orbivirus (Colorado tick fever virus), Rabies virus, Vesicular stomatitis virus, Rubivirus (rubella), Semliki Forest virus, St. Louis encephalitis virus, Venezuelan equine encephalitis virus, Venezuelan equine encephalomyelitis virus, Arenaviruses (a.k.a. South American Haemorrhagic Fever virus), Flexal, Lymphocytic choriomeningitis virus (LCM) (neurotropic strains), Hantaviruses including Hantaan virus, Rift Valley fever virus, Japanese encephalitis virus, Yellow fever virus, Monkeypox virus, Human immunodeficiency virus (HIV) types 1 and 2, Human T cell lymphotropic virus (HTLV) types 1 and 2, Simian immunodeficiency virus (SIV), Vesicular stomatitis virus, Guanarito virus, Lassa fever virus, Junin virus, Machupo virus, Sabia, Crimean-Congo hemorrhagic fever virus, Ebola viruses, Marburg virus, Tick-borne encephalitis virus complex (flavi) including Central European tick-borne encephalitis, Far Eastern tick-borne encephalitis, Hanzalova, Hypr, Kumlinge, Kyasanur Forest disease, Omsk hemorrhagic fever, and Russian Spring Summer encephalitis viruses, Herpesvirus simiae (Herpes B or Monkey B virus), Cercopithecine herpesvirus 1 (Herpes B virus), Equine morbillivirus (Hendra and Hendralike viruses), Nipah virus, Variola major virus (Smallpox virus), Variola minor virus (Alastrim), African swine fever virus, African horse sickness virus, Akabane virus, Avian influenza virus (highly pathogenic), Blue tongue virus, Camel pox virus, Classical swine fever virus, Cowdria ruminantium (heartwater), Foot and mouth disease virus, Goat pox virus, Japanese encephalitis virus, Lumpy skin disease virus, Malignant catarrhal fever virus, Menangle virus, Newcastle disease virus (VVND), Peste Des Petits Ruminants virus, Rinderpest virus, Sheep pox virus, Swine vesicular disease virus, Vesicular stomatitis virus (exotic).

The analyte can be derived from a parasite. For example, in some embodiments, the analyte is derived from a parasite selected from among Ancylostoma human hookworms including A. duodenale, A. ceylanicum, Ascaris including Ascaris lumbricoides suum, Babesia including B. divergens, B. microti, Brugia filaria worms including B. malayi, B. timori, Coccidia, Cryptosporidium including C. parvum, Cysticercus cellulosae (hydatid cyst, larva of T. solium), Echinococcus including E. granulosis, E. multilocularis, E. vogeli, Entamoeba histolytica, Enterobius, Fasciola including F. gigantica, F. hepatica, Giardia including G. lamblia, Heterophyes, Hymenolepis including H. diminuta, H. nana, Isospora, Leishmania including L. braziliensis, L. donovani, L. ethiopia, L. major, L. mexicana, L. peruvania, L. tropica, Loa loa filaria worms, Microsporidium, Naegleria fowleri, Necator human hookworms including N. americanus, Onchocerca filaria worms including, O. volvulus, Plasmodium cynomologi, P. falciparum, P. malariae, P. ovale, P. vivax, Sarcocystis including S. sui hominis, Schistosoma including S. haematobium, S. intercalatum, S. japonicum, S. mansoni, S. mekongi, Strongyloides including S. stercoralis, Taenia solium, Toxocara including T. canis, Toxoplasma including T. gondii, Trichinella spiralis, Trypanosoma including T. brucei brucei, T. brucei gambiense, T. brucei rhodesiense, T. cruzi, or Wuchereria bancrofti filaria worms.

The analyte can be a fungal pathogen. For example, in some embodiments, the analyte is derived from a fungal pathogen selected from among Aspergillus fumigates, Blastomyces dermatitidis, Cladosporium bantianum, Candida albicans, C. (Xylohypha) trichoides, Cryptococcus neoformans, Dactylaria galopava (Ochroconis gallopavum), Epidermophyton, Exophiala (Wangiella) dermatitidis, Fonsecaea pedrosoi, Microsporum, Paracoccidioides braziliensis, Penicillium marneffei, Pneumocystis carinii, Sporothrix schenckii, Trichophyton, Coccidioides immitis, Coccidioides posadasii, Histoplasma capsulatum, H. capsulatum var. duboisii.

The analyte can be a toxin. In some embodiments, the analyte is a toxin selected from among Abrin, Botulinum neurotoxins, Clostridium perfringens epsilon toxin, Conotoxins, Diacetoxyscirpenol, Ricin, Saxitoxin, Shiga-like ribosome inactivating proteins, Shigatoxin, Staphylococcal enterotoxins, T-2 toxin, and tetrodotoxin.

In some embodiments, the analyte is selected from among Hepatitis B surface antigen (HBsAg), B. burgdorferi OspA, HPV L1, RSV F protein, Influenza hamagglutanin, Influenza stem-loop region, Influenza M2, P. falciparum merozoite surface protein 1-10, GLURP, SERA, S-antigen, 6-cys family, AMA1, EBA175, 140, 181, MTRAP, PTRAMP, ASP, Rh1, 2a, 2b, 4, 5, RAP1, 2, 3, RAMA, RHOPH1, 2, 3, P. vivax circumsporozoite protein, sporozoite surface proetin2, SSP2/TRAP, CSP-N, CSP-R, CSP-C, MSP-1, MSP-9, DBPRIII, AMA-1, Pvs25, Pvs28, S. aureus capsular polysaccharide, poly-N-acetyl glucosamine, HIV gp120, gp41, and Dengue virus conserved regions.

In another embodiment the analyte comprises at least one epitope of an allergen. Allergens can be naturally occurring, or artificial such as allergens contained in allergy vaccines. Examples of allergens include, but are not limited to, animal products (for example, Fel d 1, fur dander, cockroach calyx, wool, dust mite excretion), drugs (for example, penicillin, sulfonamides, salicylates, local anaesthetic), food (for example, celery and celeriac, corn, eggs (e.g., albumin), fruit, legumes (for example, beans, peas, peanuts, soybeans), milk, seafood (e.g., shellfish), sesame, soy, tree nuts (for example, pecans, almonds), wheat, insect venom (for example, fire ants, bee sting venom, wasp sting venom), latex, metal, plant pollen (for example, grass (e.g., ryegrass, timothy-grass, weeds (e.g., ragweed, plantago, nettle, Artemisia vulgaris, chenopodium album, sorrel), and trees (e.g., birch, alder, hazel, hornbeam, aesculus, willow, poplar, platanus, tilia, olea, Ashe juniper).

In some embodiments, the analyte is an allergen derived from a latex protein, for example, unprocessed latex sap, raw latex containing ammonia, or finished latex product in which the proteins have been exposed to chemicals and high temperatures. In some embodiments, the allergen is the allergen of a mite, for example, Dermatophagoides farinae, Dermatophagoides pteronyssinus, Acarus siro, Blomia tropicalis, Chortoglyphus arcuatas, Euroglyphus cannei, Lepidoglyphus destructor, Tyrophagus putrescentiae, or Glyphagus demesticus. In some embodiments, the allergen is from venom, for example, Bombus spp., Vespa crabro, Apis mellifera, Dolichovespula spp., Polistes spp., Vespula spp., Dolichovespula maculata, or Dolichovespula arenaria. In some embodiments, the analyte is an allergen from an insect, for example, Camponotus pennsylvanicus, Solenopsis invicta, Solenopsis richteri, Periplaneta americana, Blattella germanica, Blatta orientails, Tebanus spp., Musca domestica, Ephemeroptera spp., Culicidae sp., or Heterocera spp.

In some embodiments, the allergen analyte is epithelia, dander, or hair from an organism, for example, Serinus canaria, Felis catus (domesticus), Bos taurus, Gallus gallus (domesticus), Canis familiaris, Arias platyrhynchos, Meriones unguiculatus, Capra hircus, Anser domesticus, Cavia porcellus (cobaya), Mesocrietus auratus, Sus scrofa, Equus caballus, Mus musculus, Psittacidae, Columba fasciata, Oryctolagus cuniculus, Rattus norvegicus, or Ovis aries.

In some embodiments, the allergen analyteis from fungi, for example, Cephalosporium acremonium, Alternaria tenuis, Aspergillus glaucus, Aspergillus flavus, Aspergillus fumigatus, Aspergillus nidulans, Aspergillus niger, Aspergillus terreus, Aspergillus versicolor, Aureobasidium pullulan (Pullularia pullulans), Drechslera sorokiniana, Helminthosporium sativum, Botrytis cinerea, Candida albicans, Chaetomium globosum, Cladosporium herbarum, Cladosporium sphaerospennum (Homodendrum hordei), Drechslera spicifera (Curvularia spicifera), Epicoccum nigrum (Epicoccum purpurascens), Epidermophyton floccosum, Fusarium moniliforme, Fusarium solani, Geotrichum candidum, Gliocladium viride, Helminthosporium solani, Microsporum canis, Mucor circinelloidesf circinelloides, Mucor circinelloidesf lusitanicus, Mucor plumbous, Mycogone perniciosa, Neurospora intermedia, Nigrospora oryzae, Paecilomyces variotii, Penicillum brevicompactum, Penicillum camembertii, Penicillum chrysogenum, Penicillum digitatum, Penicillum expansum, Penicillum notatum, Penicillum roquefortii, Phoma betae, Phoma herbarum, Rhizopus oryzae, Rhizopus stolonifer, Rhodotorula mucilaginosa, Saccharomyces cerevisiae, Scopulariopsis brevicaulis, Serpula lacrymans, Setosphaeria rostrata, Stemphylium botryosum, Stemphylium solani, Trichoderma harzianum, Trichophyton mentagrophytes, Trichophyton rubrum, or Trichothecium roseum. In some embodiments, the allergen is from a smut, for example, Ustilago nuda, Ustilago cynodontis, Ustilago candis, Sporisorium cruentum, Ustilago avenae, or Ustilago tritici.

In some embodiments, the allergen analyte is from a grass, for example, Paspalum notatum, Cynodon dactylon, Poa compressa, Bromus inennis, Phalaris arundinacea, Zea cans, Elytrigia repens (Agropyron repens), Sorghum haelpense, Poa pratensis, Festuca pratensis (elatior), Avena sativa, Dactylis glomerata, Agrostis gigantea (alba), Secale cereale, Leymus (Elymus) condensatus, Lolium perenne ssp. multiflorum, Lolium perenne, Anthoxanthum odoratum, Phleum pratense, Holcus lanatus, Triticum aestivum, or Elymus (Agropyron) smithii.

In some embodiments, the allergen analyte is from a weed, for example, Atriplex polycarpa, Baccharis halimifolia, Baccharis sarothroides, Hymenoclea salsola, Amaranthus hybridus, Xanthium strumarium (commune), Rumex crispus, Eupathium capillifolium, Solidago spp., Amaranthus tuberculatus (Acnida tamariscina), Allenrolfea occidentalis, Chenopodium botrys, Kochia scoparia, Chenopodium album, Iva xanthifolia, Iva angustifolia, Chenopodium ambrosioides, Artemisia vulgaris, Artemisia ludoviciana, Urtica dioica, Amaranthus spinosus, Plantago lanceolata, Iva axillaris, Atriplex lentiformis, Ambrosia dumosa, Ambrosia acanthicarpa, Ambrosia trifida, Ambrosia artemisiifolia, Ambrosia confertiflora, Ambrosia bidentata, Ambrosia psilostachya, Salsola kali (pestifer), Artemisia californica, Artemisiafrigida, Artemisia tridentata, Atriplex wrightii, Atriplex confertifolia, or Artemisia annua.

In some embodiments, the allergen analyte is from a tree, for example, Acasia spp., Alnus glutinosa, Alnus rubra, Alnus incana ssp. rugosa, Alnus rhombifolia, Fraxinus velutina, Fraxinus pennsylvanica, Fraxinus latifolia, Fraxinus americana, Populus tremuloides, Myrica cerifera, Fagus grandifolia (americana), Casuarina equisetifolia, Betula lenta, Betula pendula, Betula nigra, Betula occudentalis (fontinalis), Betula populifolia, Acer negundo, Cryptomeria japonica, Juniperus ashei (sabinoides), Juniperus virginiana, Tamarix gallica, Populus balsamifera ssp. trichocarpa, Populus deltoides, Populusfremontii, Populus wislizeni, Populus monilifera (sargentii), Cupressus arizonoca, Taxodium distichum, Cupressus sempervirens, Ulmus americana, Ulmus crassifolia, Ulmus pumila, Eucalyptus globulus, Celtis occidentalis, Corylus americana, Corylus avellana, Carya ovata, Carya laciniosa, Carya alba, Juniferus monosperma, Juniperus princhotii, Juniperus scopulorum, Juniperus occidentalis, Robinia pseudoacacia, Mangifera indica, Acer macrophyllum, Acer rubrum, Acer saccharum, Melaleuca quinquenervia (leucadendron), Prosopis glandulosa (juliflora), Broussonetia papyrifera, Morus rubra, Morums alba, Quercus gambelii, Quercus velutina, Quercus macrocarpa, Quercus kelloggii, Quercus agrifolia, Quercus lobata, Quercus ilex, Quercus stellata, Quercus rubra, Quercus dumosa, Quercus virginiana, Quercus nigra, Quercus garryana, Quercus alba, Olea europaea, Elaegnus angustifolia, Citrus sinensis, Arecastrum romanzoffianum (Cocos plumosa), Carya illnoensis, Schinus molle, Schinus terebinthifolius, Pinus taeda, Pinus strobus, Pinus palustris, Pinus ponderosa, Pinus elliottii, Pinus virginiana, Pinus monticola, Pinus echinata, Populus nigra, Populus alba, Ligustrum vulgare, Liquidambar styraciflua, Platanus occidentalis, Platanus orientalis, Platanus racemosa, Platanus acerifolia, Juglans nigra, Juglans californica, Juglans regia, Salix lasiolepsis, Salix nigra, or Salix discolor. In some embodiments, the allergen is from a flower, for example, Chrysanthemum leucanthemum, Taraxacum officinale, or Helianthus annuus. In some embodiments, the allergen is from a farm plant, for example, Medicago sativa, Ricinus communis, Trifolium pratense, Brassica spp., or Beta vulgaris.

In some embodiments, the allergen analyte is from plant food (an edible plant), for example, Prunus dulcis, Malus pumila, Prunus armeniaca, Musa paradisiaca (sapientum), Hordeum vulgare, Phaseolus lanatus, Phaseolus vulgaris, Phaseolus sp., Phaseolus sp., Phaseolus vulgaris, Rubus allegheniensis, Vaccinium sp., Brassica oleracea var. botrytis, Fagopyrum esculentum, Brassica oleracea var. capitata, Theobroma cacao, Cucumis melo, Daucus carota, Brassica oleracea var. botrytis, Apium graveolens var. dulce, Prunus sp., Cinnamomum verum, Coffea arabic, Zea cans, Vaccinium macrocarpon, Cucumis sativus, Allium sativum, Zingiber officinale, Vitis sp., Citrus paradisi, Humulus lupulus, Citrus limon, Lactuca sativa, Agaricus campestris, Brassica sp., Myristica fragrans, Avena sativa, Olea europaea, Allium cepa var. cepa, Citrus sinensis, Vigna unguiculata, Pisum sativum, Prunus persica, Pyrus communis, Piper nigrum, Capsicum annuum var. annuum, Ananas comosus, Ipomoea batatas, Solanum tuberosum, Rubus idaeus var. idaeus, Oryza sativa, Secale cereale, Sesamum orientale (indicum), Glycine max, Spinacia oleracea, Cucurbita pepo var. melopepo, Fragaria chiloensis, Lycopersicon esculentum (lycopersicum), Brassica rapa var. rapa, Vanilla planifolia, Citrullus lanatus var. lanatus, or Triticun aestivum.

In some embodiments the allergen analyte is from fish or shellfish, for example, Micropterus sp., Ictalurus punctatus, Mercenaria mercenaria, Gadus morhua, Callinectes sapidus, Platichthys sp., Hippoglossus sp., Homarus americanus, Scomber scombrus, Crassostrea virginica, Sebastes marinus, Salmo salar, Clupeiformes, Pecten magellanicus, Penaeus sp., Salvelinus sp., or Thunnus sp. In some embodiments, the allergen is an animal food product, for example, from Bos taurus, Ovis aries, or Sus scrofa. In some embodiments, the allergen is a poultry product, for example, chicken (Gallus gallus) products or turkey (Meleagris gallopavo) products. In some embodiments, the allergen is from a dairy product, for example, bovine casein or bovine milk. In some embodiments, the allergen is a nut, for example, Bertholletia excelsa, Anacardium oceidentale, Cocos nucifera, Corylus americana, Arachis hypogaea, Carya illinoensis, Juglans nigra, or Juglans regia. In some embodiments, the allergen is dust, for example, barley grain dust, corn grain dust, house dust, mattress dust, oat grain dust, wheat grain dust, upholstery dust, or latex dust.

In some embodiments, the antigen analyte is an autoantigen associated with an autoimmune disorder. In some embodiments, the autoimmune disorder is a cell or organ-specific autoimmune disorder, and the autoantigen analyte is selected from among: acetylcholine receptor (myasthenia gravis), actin (chronic active hepatitis, primary biliary cirrhosis), adenine nucleotide translocator (ANT) (dilated cardiomyoapthy, myocarditis), beta-adrenoreceptor (dilated cardiomyopathy), aromatic L-amino acid decarboxylase (autoimmune polyendocrine syndrome type I (APS-1)), asialoglycoprotein receptor (autoimmune hepatitis), bactericidal/permeability-increasing protein (Bpi) (cystic fibrosis vasculitides), calcium-sensing receptor (acquired hypoparathyroidism), cholesterol side-chain cleavage enzyme (CYPIIa) (APS-1), collagen type IV alpha3-chain (Goodpasture syndrome), cytochrome P450 2D6 (CYP2D6) (autoimmune hepatitis), desmin (Crohn disease, coronary artery disease), desmoglein 1 (pemphigus foliaceus), desmoglein 3 (pemphigus vulgaris), F-actin (autoimmune hepatitis), GM ganglioside (Guillain-Barre syndrome), glutamate decarboxylase (GAD65) (type 1 diabetes, stiff man syndrome), glutamate receptor (GLUR) (Rasmussen encephalitis), H/K ATPase (autoimmune gastritis), 17-alpha-hydroxylase (CYP17) (APS-1), 21-hydroxylase (CYP21) (Addison disease), IA-2 (ICA512) (type 1 diabetes), insulin (type 1 diabetes, insulin hypoglycemic syndrome (Hirata disease), type B insulin resistance, acanthosis, systemic lupus erythematosus (SLE)), intrinsic factor type 1 (pernicious anemia), leukocyte function-associated antigen (LFA-1) (treatment-resistant lyme arthritis), myelin-associated glycoprotein (MAG) (polyneuropathy), myelin basic protein (multiple sclerosis, demyelinating disease), myelin oligodendrocyte glycoprotein (MOG) (multiple sclerosis), myosin (rheumatic fever), p-80-Coilin (atopic dermatitis), pyruvae dehydrogenase complex-E2 (PDC E2) (primary biliary cirrhosis), sodium iodide symporter (NIS) (Graves disease, autoimmune hypothyroidism), SOX-10 (vitiligo), thyroid and eye muscle shared protein (autoimmune thyroiditis), thyroid peroxidase (autoimmune Hashimoto thyroiditis), thyrotropin receptor (Graves disease), tissue transglutaminase (celiac disease), transcription coactivator p75 (atopic dermatitis), tryptophan hydroxylase (APS-1), tyroisinase (vitiligo, metastatic melanoma), and tyrosine hydroxylase (APS-1), wherein the associated autoimmune disorder(s) is listed parenthetically immediately after each autoantigen analyte.

In some embodiments, the autoimmune disorder is a systemic autoimmune disorder, and the autoantigen analyte is selected from among: ACTH (ACTH deficiency), aminoacyl-tRNA histidyl synthetase (myositis, dermatomyositis), aminoacyl-tRNA synthetase (polymyositis, dermatomyositis), cardiolipin (SLE), carbonic anhydrase II (SLE, Sjogren syndrome, systemic sclerosis), collagen (rheumatoid arthritis (RA), SLE, progressive systemic sclerosis), centromere-associated protein (systemic sclerosis), DNA-dependent nucleosome-stimulated ATPase (dermatomyositis), fibrillarin (scleroderma), fibronectin (SLE, RA, morphea), glucose-6-phosphate isomerase (RA), Beta2-glycoprotein I (Beta2-GPI) (primary antiphospholipid syndrome), golgin (95, 97, 160, and/or 180) (Sjogren syndrome, SLE, RA), heat shock protein (various immune related disorders), hemidesmosomal protein 180 (bullous pemphigoid, herpes gestationis, cicatricial pemphigoid, histone H2A-H2B-DNA (SLE), IgE receptor (chronic idiopathic urticaria), keratin (RA), Ku-DNA-protein kinase (SLE), Kunucleoprotein (connective tissue syndromes), La phosphoprotein (La 55-B) (Sjoren syndrome), myeloperoxidase (necrotizing and cescentic glomerulonephritis (NCGN), system vasculitis), proteinase 3 (PR3) (Wegener granulomatosis, Churg-Strauss syndrome), RNA polymerase I-III (RNP) (systemic sclerosis, SLE), signal recognition protein (SRP54) (polymyositis), topoisomerase-1 (Scl-70) (scleroderma, Raynaud syndrome), tubulin (chronic liver disease, visceral leishmaniasis), and vimentin (systemic autoimmune disease), wherein the associated autoimmune disorder(s) is listed parenthetically immediately after each autoantigen.

In some embodiments, the autoimmune disorder is a plasma protein autoimmune disorder or cytokine autoimmune disorder, and the autoantigen analyte is selected from among: C1 inhibitor (autoimmune C1 deficiency), C1q (SLE, membrane proliferative glomerulonephritis (MPGN)), cytokine (e.g., IL-1 alpha, IL-1beta, IL-, IL-10, LIF) (RA, systemic sclerosis), factor II (prolonged coagulation time), factor V (prolonged coagulation time), factor VII (prolonged coagulation time), factor VIII (prolonged coagulation time), factor IX (prolonged coagulation time), factor X (prolonged coagulation time), factor XI (prolonged coagulation time), factor XII (prolonged coagulation time), thrombin (prolonged coagulation time), vWF (prolonged coagulation time), glycoprotein IIb/IIIg and Ib/IX (autoimmune thrombocytopenia purpura), IgA (immunodeficiency), and oxidized LDL (OxLDL) (atherosclerosis), wherein the associated autoimmune disorder(s) is listed parenthetically immediately after each autoantigen analyte.

In some embodiments, the autoimmune disorder is a cancer or paraneoplastic autoimmune disorder, and the autoantigen analyte is selected from among: amphiphysin (neuropathy, small lung cell cancer), cyclin B 1 (hepatocellular carcinoma), DNA topoisomerase II (liver cancer), desmoplakin (paraneoplastic pemphigus), gephyrin (paraneoplastic stiff man syndrome), Hu protein (paraneoplastic encephalomyelitis), neuronal nicotinic acetylcholine receptor (subacute autonomic neuropathy, cancer), p53 (cancer, SLE), p62 (IGF-II mRNA-binding protein) (hepatocellular carcinoma), recoverin (cancer-associated retinopathy), R1 protein (paraneoplastic opsoclonus myoclonus ataxia), beta IV spectrin (lower motor neuron syndrome), synaptotagmin (Lambert-Eaton myasthenic syndrome), voltage-gated calcium channels (Lambert-Eaton myasthenic syndrome) and Yo protein (paraneoplastic cerebellar degeneration).

In some embodiments, the antigen analyte is an endogenous antigen that is an aberrantly expressed polypeptide. Examples of such endogenous antigens include, but are not limited to, amyloid beta (A-beta), alpha synuclein, cystatin C, tau, ABri, ADan, superoxide dismutase (SOD), mutant Huntington, PrP^{sc} or a fragment of any of the foregoing.

In some embodiments of the invention, the analyte comprises at least one epitope of an implant to be introduced into a subject, metabolic or degradation products of an implant material, or substances that specifically bind to an epitope of an implant material, such as antibodies developed to an implant material or its degradation products Such implants can include, for example, electrically powered implants (for example, artificial pacemakers), bioimplants (biomaterial surgically implanted in a subject's body to replace damaged tissue (for example, orthopedic reconstructive prosthesis), cardiac prostheses (artificial valves), skin, and cornea), contraceptive implants, dental implants, orthopedic implants, and adhesion prevention devices. Examples of implant materials that can bear epitopes include latex; silicone; metals, such as cobalt chrome (Co--Cr) alloys, titanium, and titanium alloys; polymers, such as ultrahigh molecular weight polyethylene (UHMWPE) and polymethyl methacrylate cement (PMMA); and bioceramics, such as hydroxyapatite and Bioglass.

In certain embodiments, the non-antibody binding element can be a bacterial binding protein, or an antibody binding domain. The predetermined analyte can be selected from beneficial gut bacteria, pathogenic bacteria, protein toxins, protein biomarkers, small molecule toxins, metabolites, or chemical warfare agents. For such analytes, in some embodiments, the assay is modified into a competitive format, wherein the small molecule analyte is linked to a protein or other macromolecular carrier in such a way that antibodies to the free analyte also recognize the immobilized analyte (if no antibodies are available they can be generated by immunizing animals with the immobilized analyte). The immobilized analyte will aggregate cell receptors that are bound to the anti-analyte antibody and give a luminescent signal. If the immobilized analyte is mixed with the free analyte to be measured, which cannot lead to aggregation, the luminescent signal will be reduced. The activator can be a receptor and the non-antibody binding element can be a ligand that is specific for the receptor and causes a conformational change (rather than aggregation) in the receptor when bound thereto, wherein the ligand is adapted to bind to the receptor only after it has bound to the predetermined analyte. The ligand can be fused to a detector, wherein the detector is operative to prevent the ligand from binding to the receptor unless the ligand has first bound to the predetermined analyte. The activator can also be a receptor that has been engineered to bind a predetermined analyte, wherein the receptor undergoes a conformational change upon binding the predetermined analyte. Again, this variant does not rely on an aggregation effect. However, in other embodiments, an aggregation event can be mediated by a carrier molecule such as serum albumin that binds multiple copies of a target such as physiological or drug metabolites.

### Example Biosensor I (for reference purposes)

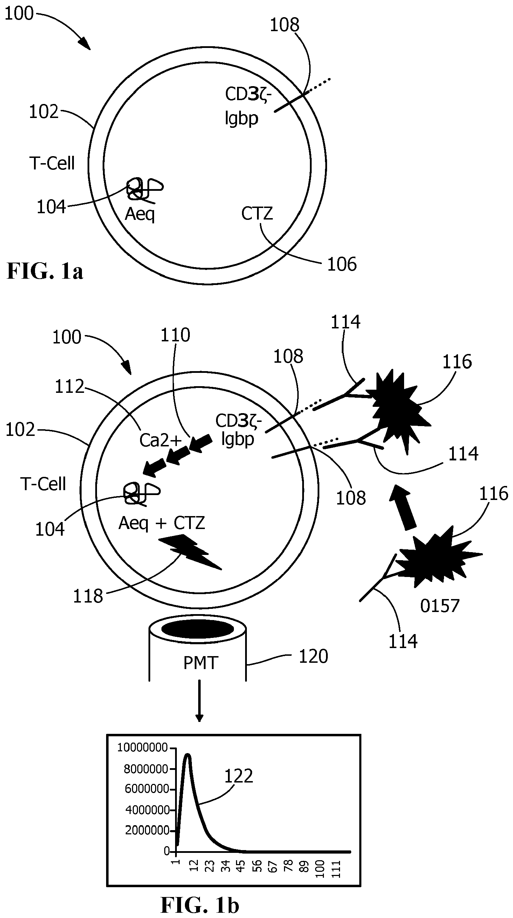

With reference to FIGS. 1a-b, a first biosensor 100 includes Jurkat T cells 102 that have been engineered to produce aequorin 104 and that have been charged with CTZ 106 to form an aequorin/CTZ complex, as previously described. This particular biosensor has also been engineered to express the transmembrane non-antibody signal transducing element 108, which is IgGbp-CD3ζ (SEQ ID NOS: 5-6), although the transmembrane non-antibody signal transducing element FcγRI-CD3ζ (SEQ ID NO: 11-12) may also be used with biosensor 100. Biosensor cell 102 also includes at least one signal transduction pathway 110, the activation of which results in an increase of intracellular Ca2+ 112. As shown in FIG. 1b, when a sufficient number of detector molecules 114 (e.g., soluble antibodies) to which target analyte 116 (e.g., *E. coli* 0157) is bound bind to transmembrane non-antibody signal transducing elements 108, signal transduction pathway 110 is activated, intracellular Ca2+ 112 increases, the aequorin/CTZ complex undergoes a conformational change and emits a signal (photon) of light 118 which is detected by photo multiplier tube 120, and spike 122 is graphically displayed on a testing device (see description below), indicating the presence of target analyte 116 within a sample being tested. The display may be both qualitative and quantitative with regard to target analyte 116.

### Example Biosensor II (for reference purposes)

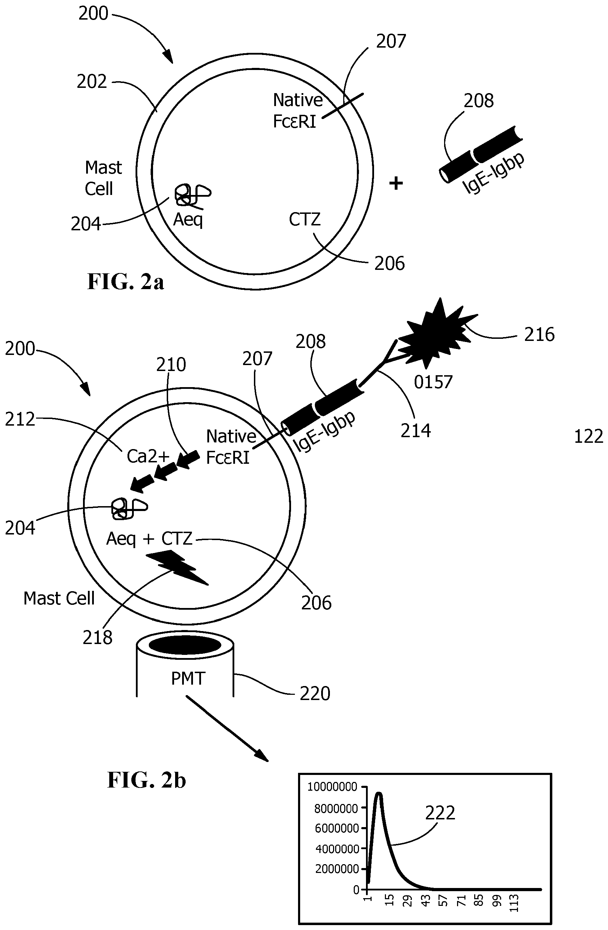

With reference to FIGS. 2a-b, a second biosensor 200 includes MC/9 (ATCC^{®} CRL-8306^{™}) mast cells 202 that have been engineered to produce aequorin 204 and that have been charged with CTZ 206 to form an aequorin/CTZ complex, as previously described. This particular biosensor expresses the native Fc epsilon receptor (i.e., FcεRI) 207, which binds to soluble non-antibody signal transducing element 208, which is IgGbp-IgE (SEQ ID NOS: 13-14), although the non-antibody signal transducing element FcyRI-IgE. (SEQ ID NO: 15-16) may also be used with biosensor 200. As shown in FIG. 2b, when a sufficient number of detector molecules 214 (e.g., soluble antibodies) to which target analyte 216 (e.g., *E. coli* 0157) is bound bind to non-antibody signal transducing elements 208 that have previously bound to native Fc epsilon receptors 207, signal transduction pathway 210 is activated, intracellular Ca2+ 212 increases, the aequorin/CTZ complex undergoes a conformational change and emits a signal (photon) of light 218 which is detected by photo multiplier tube 220, and spike 222 is graphically displayed on a testing device (see description below), indicating the presence of target analyte 216 within a sample being tested. The display may be both qualitative and quantitative with regard to target analyte 216.

### Example Biosensor III (for reference purposes)

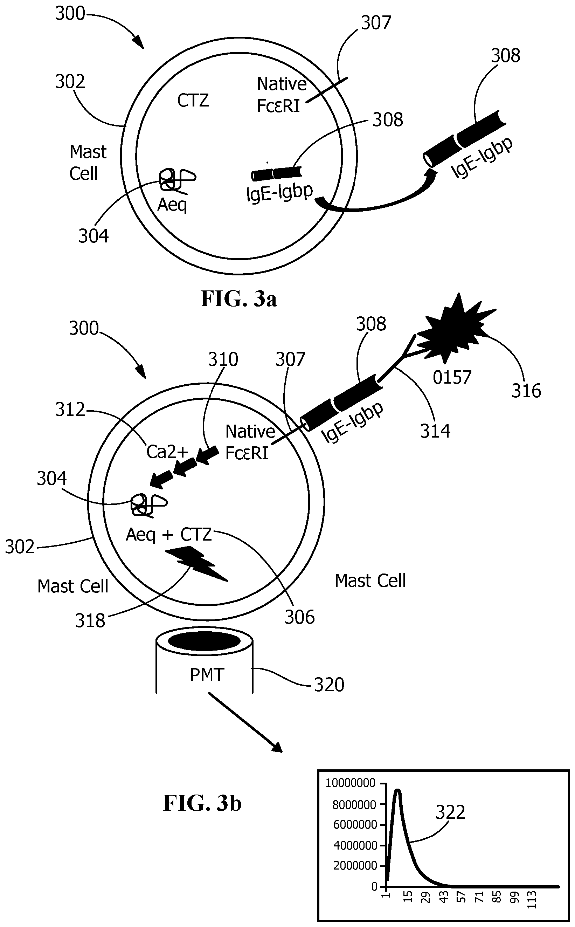

With reference to FIGS. 3a-b, a third biosensor 300 includes MC/9 (ATCC^{®} CRL-8306^{™}) mast cells 302 that have been engineered to produce aequorin 304 and that have been charged with CTZ 306 to form an aequorin/CTZ complex, as previously described. This particular biosensor expresses the native Fc epsilon receptor (i.e., FcεRI) 307, which binds to non-antibody signal transducing element 308, which is IgGbp-IgE (SEQ ID NOS: 13-14), although the non-antibody signal transducing element FcyRI-IgE. (SEQ ID NO: 15-16) may also be used with biosensor 300. In this particular example, biosensor cells 302 have been further engineered to express IgGbp-IgE and excrete this non-antibody signal transducing element into the extracellular space, wherein it binds to the native FcεRI expressed on the cell surface. As shown in FIG. 3b, when a sufficient number of detector molecules 314 (e.g., soluble antibodies) to which target analyte 316 (e.g., *E. coli* 0157) is bound bind to non-antibody signal transducing elements 308 that have previously bound to native Fc epsilon receptors 307, signal transduction pathway 310 is activated, intracellular Ca2+ 312 increases, the aequorin/CTZ complex undergoes a conformational change and emits a signal (photon) of light 318 which is detected by photo multiplier tube 320, and spike 322 is graphically displayed on a testing device (see description below), indicating the presence of target analyte 316 within a sample being tested. The display may be both qualitative and quantitative with regard to target analyte 316.

### Example Biosensor IV (for reference purposes)

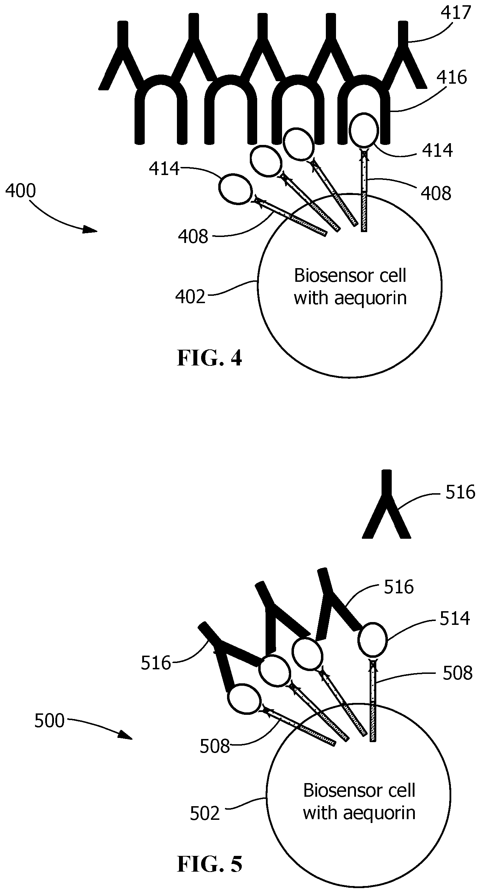

With reference to FIGS. 4, a fourth biosensor 400 includes biosensor cells 402 that have been engineered to produce aequorin and to express transmembrane non-antibody signal transducing element 408, which is mSA-CD3ζ (SEQ ID NO: 17-18). Non-antibody signal transducing element mSA-CD3ζ (monomeric streptavidin-CD3ζ) binds to a biotinylated detector molecule 414, which specifically binds to a target molecule 416 such as, for example, epidermal growth factor (EGF). An anti-target molecule antibody 417 such as, for example, anti-EGF, creates target multimers that cluster multiple signal transducing elements and induce signal transduction as previously described. In other instances, the monomeric streptavidin component is replaced with a biotinylated component and alternate linkage means may be employed.

### Example Biosensor V (for reference purposes)

With reference to FIG. 5, a fifth biosensor 500 includes biosensor cells 502 that have been engineered to produce aequorin and to express transmembrane non-antibody signal transducing element 508, which is mSA-CD3ζ (SEQ ID NO: 17-18). Non-antibody signal transducing element mSA-CD3ζ (monomeric streptavidin-CD3ζ) binds to a biotinylated detector molecule 514, which may be an autoantigen molecule. Biotinylated detector molecule 514 specifically binds to a target molecule 516, which may be an anti-autoantigen molecule. Autoantibodies in a serum sample create target multimers that cluster multiple signal transducing elements and induce signal transduction as previously described. In other instances, the monomeric streptavidin component is replaced with a biotinylated component and alternate linkage means may be employed.

The amino acid sequences of the signal-transducing polypeptide used to produce the chimeric proteins disclosed herein may have at least 70%, 75%, 80%, 85%, 87.5%, 90%, 92.5%, 95%, 97.5%, 98%, 99% sequence identity or similarity to the proteins or domains identified by or in the following accession numbers: IgM heavy chain (GenBank: CAC20458.1), Ig-alpha (P11912.2, GI:547896), Ig-beta (P40259.1 GI:728994), CD19 (AAA69966.1 GI:901823), CD3zeta (P20963.2, GI: 23830999), IgE alpha (1F2Q_A, GI:9257150) and Fc-epsilonR1 subunit alpha (P12319.1, GI: 119865).

*Staphylococcus aureus* Protein A (P02976.3, GI: 110283003) is encoded by the *spa* gene of *Staphylococcus aureus* and its structure, including its Ig-binding segments, and immunoglobulin-binding properties are well-known and are disclosed in Graille, et al., Proc Natl Acad Sci U S A. 2000 May 9;97(10):5399-404; and Roben, et al. J Immunol. 1995 Jun 15;154(12):6437-45. Variants of Protein A or its immunoglobulin-binding segments having at least 70%, 75%, 80%, 85%, 87.5%, 90%, 92.5%, 95%, 97.5%, 98%, 99% sequence identity or similarity to known Protein A amino acid sequences and the capacity to bind to an immunoglobulin or other analyte, such as those described by Graille, *et al.* and Roben, *et al.*, may be produced by molecular biological techniques well-known in the art including by direct synthesis of a nucleic acid encoding an immunoglobulin-binding amino acid sequence.

Other bacterial immunoglobulin-binding proteins, such as *Streptococcus* Protein G and engineered variants of such proteins, are known and are disclosed in Bailey, et al., J Immunol Methods. 2014 Dec 15; 415:24-30 (doi: 10.1016/j.jim.2014.10.003) (Epub 2014 Oct 22); and to Watanabe, et al., J Biol Chem. 2009 May 1; 284(18): 12373-8 (doi: 10.1074/jbc.M809236200)(Epub 2009 Mar 6). Variants of Protein G or its immunoglobulin-binding segments having at least 70%, 75%, 80%, 85%, 87.5%, 90%, 92.5%, 95%, 97.5%, 98%, 99% sequence identity or similarity to known Protein G amino acid sequences and the capacity to bind to an immunoglobulin or other analyte, such as those described by Bailey, *et al.* and Watanabe, *et al.* may be produced by molecular biological techniques well-known in the art including by direct synthesis of a nucleic acid encoding an immunoglobulin-binding amino acid sequence.

Fc receptors (FcR) bind to the Fc portion of an immunoglobulin and many types such Fc receptors are known, including FcyRI and FcεRI. The structural and functional binding characteristics of these FcRs are disclosed in Fridman, FASEB J. 1991 Sep; 5(12):2684-90. Variants of FcRs or their immunoglobulin-binding segments having at least 70%, 75%, 80%, 85%, 87.5%, 90%, 92.5%, 95%, 97.5%, 98%, 99% sequence identity or similarity to a known FcR amino acid sequence, such as a sequence described by Fridman, may be produced by molecular biological techniques well-known in the art including by direct synthesis of a nucleic acid encoding an immunoglobulin-binding amino acid sequence.

A signal transducing protein disclosed herein may have at least 70%, 75%, 80%, 85%, 87.5%, 90%, 92.5%, 95%, 97.5%, 98%, 99% sequence identity or similarity to the disclosed chimeric signal transducing proteins described by SEQ ID NOS: 2, 4, 6, 8, 10, 12, 14, 16, and 18 and also have the ability to bind an analyte, such as an immunoglobulin, and then transduce a signal into the engineered biosensor cell. Such variants may be constructed by methods well known in the molecular biological arts or by chemical synthesis of polynucleotides encoding the variant chimeric reporter proteins, insertion of the encoding sequences into a vector, and transformation or transfection of a competent cell with the vector.

### Cell Sorting and Cloning

The design and construction of the biosensors of this invention resulted in a mixed population of biosensor cells when cultured. Some cells did not express the engineered factors while others expressed the factors at varying levels. Following successful electroporation and gene insertion, biosensor cells were cultured and tested for biological response (flash signal) as mixed populations. Single cell sorting was performed using a Flow Cytometer. Cells were isolated and then expanded for analysis to select those that expressed high levels of the desired proteins. For this process, fluorescently labeled antibodies were used to target different receptors on the biosensor cells, thereby enabling the sorting process. Individual clones were screened for signaling and the best clones were selected. Through this process, the most suitable clones were identified and isolated. Fluorescence-Activated Cell Sorting (FACS) and live cell staining for extracellular protein was conducted as described below.

Engineered biosensor cells were counted, gently centrifuged, and re-suspended in wash buffer (HBSS + 2% BSA) to a final concentration of 1×10⁷-1×10⁸ cells/mL. In each experiment, either a full antibody with the Fc region or F(ab)₂ was used. When using the full antibody, 1-0.5 µg of Fc receptor blocking antibody was added to each empty 12 × 15 mm tube that was to receive cells. Into each of these tubes, 100 µL of cells (1×10⁶ to 1×10⁷ cells) was added on top of the Fc blocking antibody. Cells were mixed gently and incubated for 15 minutes at 4°C or room temperature. When using F(ab)₂, the previous step of blocking Fc was omitted. A total of 1 µg of primary antibody (against the receptor of choice) was added, and the cells were then mixed gently before incubating for 20-40 minutes on ice (or at 4°C). This temperature prevented receptor internalization. Cells were gently agitated (swirled) intermittently to encourage labeling. A 2 mL volume of cold wash buffer was added then cells were centrifuged at 4°C and the supernatant discarded. The wash step was repeated before re-suspending cells in 100 µL of wash/sorting buffer. A secondary FITC-labeled antibody was added (0.5-1 µg) to the cells and mixed before incubating on ice (or at 4°C) for 20-40 minutes. Cells were protected from light during the entire process. A 2 mL volume of cold wash buffer was added then cells centrifuged and supernatant discarded. The wash step was repeated and cells re-suspend in 0.5-1 mL of wash buffer. Cells were incubated on ice until the time for sorting. Sorting was done as soon as possible (at least on the same day). Cloning and culturing cells after single cell sorting was conducted as described below.

Biosensor cells were sorted into 96 well plates with each well containing one cell and 100-200 µL cell growth media. Plates were scanned/monitored for the next 10 to 14 days to determine the rate of growth and to judge when to transfer to a 24 well plate. During scanning, different markings were used for different conditions. Some wells were marked if they contained live cells, but were not ready for transfer and contaminated wells were also marked. Cells were transferred to a 24 well plate containing 1.0 mL of the appropriate media in each well. In cases of contaminated cells, washing was done by adding all of the cell suspension from a well into 5ml of sterile 1x PBS in a 15mL conical tube. Cells were then centrifuged at 170 RCF for 10 minutes and the supernatant discarded. The pellet was re-suspended in 1.0 mL of fresh media in a 24 well plate to be cultured. Following continued growth, cells were transferred to a 12 well plate with 1.5 mL of fresh media per well.

For clone screening, after growing in a 12 well plate, cells were counted to determine if they were ready for charging and flash testing. During flash testing, a single iteration involved 25,000 cells. Enough cells were grown to accommodate testing and also leave some to continue growing. This step marked the first round of clone screening. Selected clones were grown further and subjected to subsequent tests depending on the desired properties. For biological response, for example, Jurkat-FcγRI-CD3ζ clones were screened using anti-CD3ε antibodies (positive control) and monoclonal antibodies against bacteria with the respective bacteria while Digitonin was used for chemical response test. MC/9-Aeq clones were screened using anti-FcεRI antibodies (biological response) and Digitonin (chemical response).

In summary, fluorescence-activated cell sorting (FACS) was performed using fluorescent antibody labels to select and isolate cells which were highly expressing the desired proteins, which in this case were the engineered receptors. This process resulted in a population of highly-expressing biosensor cells which was further confirmed by flash testing using the PMT in the testing device. During the entire process, cells were counted using an automatic cell counter to eliminate human error and enhance consistency and efficiency. Different clones of Jurkat-FcγRI-CD3ζ gave different levels of biological response when tested with Anti-E. *coli* O111 mAb and *E. coli* O111 bacteria. Many clones were tested the same way and the highest responders were saved in a clone bank. Likewise, chemical response results obtained from testing different MC/9-Aeq clones using the chemical Digitonin indicated that different clones gave different levels of chemical response depending on the level of Aequorin expression. The clones with the highest signal were saved in the clone bank.

### Culturing Biosensor Cells

Different culture media formulations were used for different cell lines to ensure optimal growth conditions. MC/9 mast cells were cultured in Complete Mast Cell Media (DMEM - Sigma, Cat. No. D5796; 1x Pen/Strep; 10% FBS; 10% T-Stim Supplement; 50 µM β-mercaptoethanol). Jurkat T-cells were cultured in complete RPMI media (RPMI-ThermoFisher; 10% FBS; 1x Pen/Strep). Depending on the characteristics of the electroporated constructs, different antibiotics were used for selection in cell culture. Appropriate antibiotics were added to the growth media 2-3 days after electroporation to select for cells that had successfully integrated the linearized DNA constructs. Cell concentration was kept between 4.0 × 10⁵ and 1.0 × 10⁶ cells/mL for optimal cell growth. Different cell lines and clones were processed for long term storage and stocks were frozen in liquid nitrogen as follows: (i) cells were centrifuged at 150 RCF for 10 minutes and the supernatant was discarded; (ii) the cell pellet was re-suspended in freezing media (RPMI; 50% FBS; 10% DMSO) at a concentration of 5.0 × 10⁵ cells/mL; and (iii) volumes of 1 mL were aliquoted into 2 mL Nunc Cryo-vials and frozen at -80°C for 24 hours before being transferred to liquid nitrogen for long term storage.

### Charging Biosensor Cells

The biosensor cells of this invention were centrifuged in 50 mL conical tubes at 150 RCF for 10 minutes. The supernatant was discarded and pellet re-suspended in charging media (RPMI; 10% antibody-depleted FBS; 1x pen/strep; 0.1% Pluronic F68 and 1.5 mM coelenterazine) at a concentration of 25,000 cells/180 µL. Additionally, cells were also charged at different concentrations such as, for example, 100,000 cells/180 µL and 400,000 cells/180 µL. Cells were charged at room temperature with gentle shaking/rocking for 24-26 hours. Before use, the commercially available antibody-depleted FBS may be purified further using other antibody depletion systems.

### Concentrating Cells

The biosensor cells of this invention were demonstrated to be more effectively charged at lower concentrations rather than higher concentrations. For example, charging cells at a density of 25,000 cells/180 µL versus 400,000 cells/180 µL was shown to result in a two-fold increase in detectable signal. Jurkat-FcγRI-CD3ζ clone P5G7 cells were charged at both 400,000 cells/180 µL and 25,000 cells/180 µL then tested at 400,000 cells/180 µL in each reaction. Overnight *E. coli* O111 bacteria culture was used with 23 nM anti-*E*. *coli* O111 mAb. Biosensor cells charged at a lower concentration gave a higher signal for the same number of bacteria cells tested. Biosensor cell density is mostly changed by concentrating the cells after charging to allow for optimal pathogen detection. Different concentrations were used for pathogen detection depending on the target pathogen and quality of the antibody, when the detector molecule is a soluble antibody. Biosensor cells are concentrated by centrifugation at 150 RCF for 10 minutes and the cell pellet is re-suspended in the desired volume of the testing medium. The charging medium may also serve as the testing medium. In certain instances, addition of normal FBS to the media triggered a biological response resulting in a biosensor signal (flash) due to high concentration of antibodies in normal FBS. Therefore, commercially available antibody-depleted FBS was used in the charging process, which reduced the antibody triggered signal without totally eliminating it. Additional methods were used to further deplete traces of antibodies in the commercially available antibody-depleted FBS.

### Bioassay

In exemplary embodiments of this invention, the analyte bioassay is formatted with the biosensor cell and a soluble monoclonal antibody (mAb) that is specific for that analyte (e.g., pathogen). In these embodiments, the specificity of the bioassay is directly related to the selective binding of the soluble antibody to the target analyte and the specificity and sensitivity of the biosensor is determined by detection and measurement of bioluminescence. In this process, biosensor cells are initially charged using the light-emitting molecule, coelenterazine (CTZ). The soluble antibody of choice and the sample being analyzed are then added. If a target pathogen is present in the sample, it interacts with the soluble antibody, which binds to a fusion protein expressed by the biosensor cell, ultimately triggering a signal cascade that results in light emission from the biosensor cell. The emitted light is detected by a photo multiplier tube (PMT) in the testing device and the signal emitted by the biosensor cell is displayed as photon counts per second. As described below, various methods have been developed to detect pathogens based on the soluble antibody and the target pathogen. Three such methods, described in detail below, include: (i) instant addition of detector molecules (e.g., antibodies); (ii) coating biosensor cells with detector molecules (e.g., antibodies); and (iii) coating analytes (e.g., bacteria) with antibodies.

### Testing Unit

The bioassay aspect of the present invention herein may be carried out in a testing subunit or test cartridge designed for use with a bench-top or portable testing system and device such as that disclosed in U.S. Patent No. 9,023,640. The test cartridge, which may be a single-use, disposable item, receives both the sample and the biosensor and introducing the biosensor into the test cartridge mixes the sample and the biosensor in a predictable and controlled manner. The test cartridge further includes a reaction chamber for receiving the test sample and the biosensor, wherein the reaction chamber has a predetermined internal geometry and has been further adapted to minimize or eliminate background noise for the purpose of improving the overall signal to noise ratio. At least one stabilizer may be located in the reaction chamber for minimizing shear force damage to the test sample and biosensor during the mixing process.

In an exemplary embodiment, the reaction chamber and fluid channels that lead to the reaction chamber within the test cartridge are designed to achieve several objectives. An inlet channel for fluid entering the reaction chamber includes a tubular shape and the diameter of the tube is relatively small and tapers to become smaller at the inlet to the reaction chamber. This increases the velocity of fluid entering the reaction chamber and promotes more vigorous and homogenous mixing due to the bulk motion of the reagents within the reaction chamber. It is desirable to mix the reagents and sample in a way to promote mixing beyond molecular diffusion, in order to minimize the duration of the test by ensuring that any infectious agent present in the sample rapidly encounters the biosensor. The inlet channel may be offset from the central axis of the reaction chamber to promote a clockwise or counterclockwise rotational motion of the reagents around the central axis of the test chamber as the fluids are mixed in order to increase homogeneity of the mixture. The inlet channel is also approximately tangent to the interior surface of the reaction chamber for allowing incoming fluid to travel from the inlet channel to the reaction chamber while remaining in contact with the side surface of the reaction chamber, which allows for a minimally turbulent flow and minimal introduction of air bubbles into the mixed fluids. Bubbles are undesirable due to the unpredictable refraction of light they cause as light emitted by the reagents travels through bubbles within the mixed reagents or on the surface of the mixed reagents. The axis of the inlet channel may be angled above horizontal (e.g., about 30 degrees) to provide a partially downward direction to the incoming fluid flow to ensure that the reagent is mixed with the fluid residing at the bottom of the reaction chamber. Alternatively, the reagents may be introduced to the test chamber using alternative fluid delivery means such as a vertical channel to deliver the reagents to the bottom of the reaction chamber, or delivering the fluid directly on the central axis of the test chamber in order to create a column of reagent flowing into the test chamber thereby promoting mixing through entrainment.

The shape (i.e., predetermined geometry) of the reaction chamber may be a revolved section facilitating clockwise or counterclockwise motion of the mixing fluids around the central axis of the reaction chamber. Alternatively, if desired, a reaction chamber shape other than a revolved section such as a rectangular or irregular shape may be utilized. In one embodiment, the revolved section used to form the reaction chamber is a portion of an ellipse for facilitating the collection of stray light emitted by the reagents and reflecting this light toward the surface of the detector, which may be a photomultiplier tube (PMT) (Hamamatsu). The surface of the reaction chamber may be reflective, in order to enhance the light collection properties of the elliptical shape. In some embodiments, the maximum diameter of the surface of the PMT is limited to achieve a maximum signal to noise ratio of the output of the system. The diameter of the reaction chamber may be designed to approximately match the diameter of the PMT, which influences the elliptical shape that can be achieved in a reaction chamber designed to hold a specific volume of fluids. Due to the constrained elliptical shape, the reaction chamber surface color may be a partially diffusing white due to the additional light collection that occurs when light that would not otherwise be reflected directly to the PMT surface is partially diffused by the white surface and a fraction of this is directed toward the PMT surface. Alternatively, other surface finishes and materials such as a near-mirror finish aluminum, or a transparent material could be used if desired. Further, it is desirable for the reaction chamber material to be minimally phosphorescent, in order to prevent light emitted from the reaction chamber itself from eclipsing any emitted light from the reagents and preventing detection. Although white polymeric materials such as acrylonitrile butadiene styrene or other such polymeric materials have been found to exhibit a low level of phosphorescence, the additional light collection provided by the combination of light reflection and diffusion has been found to be a benefit to the signal to noise ratio of the light sensing circuit output.

In an exemplary embodiment, the testing subunit provides a system for use in sample analysis. The system includes a biosensor reagent, wherein the biosensor reagent includes living biological cells; a reservoir card, having a long loop portion and a short loop portion, wherein the reservoir card stores the biosensor reagent; and a test cartridge base, wherein the test cartridge base is configured to accept the reservoir card. The test cartridge base further includes: (i) a reaction chamber having a central axis, wherein the reaction chamber has the shape of a revolved half ellipse; and (ii) an inlet channel connected to the reaction chamber, wherein the inlet channel is positioned above the reaction chamber at an angle of 15-60 degrees above the horizontal, wherein the inlet channel is offset from the central axis of the reaction chamber, and wherein upon introducing a sample to be analyzed into the test cartridge base through the inlet channel, the sample is homogeneously mixed with the biosensor reagent while minimizing damage to the living biological cells.

In another exemplary embodiment, the testing subunit provides a system for rapidly detecting the presence of an analyte in a biological sample. This system includes a biosensor reagent including at least one antibody specific for a predetermined analyte and a bioluminescent agent, wherein the at least one antibody is expressed on the surface of living, engineered lymphocytes and wherein the bioluminescent agent is expressed by the living, engineered lymphocytes, the biosensor reagent being operative to: (i) detect the presence of a specific analyte in a sample to be tested, and (ii) emit a detectable light signal when the biosensor reagent reacts with the sample and detects the presence of the specific analyte in the sample. A test cartridge is also included. The test cartridge further includes: (i) a reservoir card, wherein the reservoir card further includes the biosensor reagent; and (ii) a test cartridge base, wherein the test cartridge base is configured to accept the reservoir card. The test cartridge base further includes: a) a reaction chamber having a central axis, wherein the reaction chamber has the shape of a revolved half ellipse; b) an inlet channel connected to the reaction chamber, wherein the inlet channel is positioned above the reaction chamber at an angle of 15-60 degrees above the horizontal, and wherein the inlet channel is offset from the central axis of the reaction chamber; and c) wherein upon introducing the sample into the test cartridge base through the inlet channel, the sample is homogeneously mixed with the biosensor reagent while minimizing damage to the living, engineered lymphocytes and minimizing any bubbling of the mixed biosensor reagent and sample in the reaction chamber. A testing unit adapted to receive the test cartridge is also included. The testing unit including a sensor for detecting the detectable light signal emitted by the biosensor reagent upon reacting with the sample, the detection of the emitted detectable light signal being indicative of the presence of the analyte in the sample and, wherein detection of the specific analyte in the sample occurs in real time.

### Example Bioassay 1: Instant Addition of Antibodies

In an exemplary embodiment of the bioassay of the present invention, wherein the detector molecule is a soluble antibody, the soluble antibody and sample to be tested are mixed together immediately prior introduction of the biosensor cells to the test sample. In this embodiment, charged biosensor cells are centrifuged and concentrated to about 400,000 cells/180 µL (adequate for a single reaction) in the charging medium. A 180 µL (about 400,000 cells) aliquot of the charged biosensor cells is then loaded into the long loop portion of the reservoir card. For a positive control, 30 µL of anti-CD3ε antibody in RPMI media is loaded into the short loop portion of the reservoir card. The reservoir card is then locked into the test cartridge base. A 2 µL volume of an antibody (at 0.5 mg/mL) against the target pathogen, such as anti-E. *coli* O111 (wherein the target pathogen is *E. coli* O111), is mixed with 28 µL of the sample to be tested in the cartridge mixing chamber. The test cartridge base is inserted into the testing device and the charged biosensor cells are injected into the reaction chamber to initiate the reaction. The resulting signal is recorded for 4 to 8 minutes and at the end of the test period, the 30 µL of anti-CD3ε antibody is injected from the short loop of the reservoir into reaction chamber as a positive control reaction that is recorded for 2 minutes. As an alternative positive control, 30 µL of 0.61 mM Digitonin can be used rather than anti-CD3ε antibody. A negative control test can be performed using a predetermined pathogen that is not specific for the antibody being used.

### Example Bioassay 2: Coating Biosensor Cells with Antibodies

In another exemplary embodiment of the bioassay of the present invention, wherein the detector molecule is a soluble antibody, the biosensor cells are coated with the soluble antibody for a period of time prior to mixing the sample to be tested with the biosensor cells. In this embodiment, charged biosensor cells are centrifuged and concentrated to about 400,000 cells/180 µL (adequate for one reaction) in the charging medium. A 180 µL (about 400,000 cells) aliquot of the biosensor cells is then mixed with a 2 µL volume of an antibody (at 0.5 mg/mL) against the target pathogen, such as anti-E. *coli* O111 (wherein the target pathogen is *E. coli* O111), in an Eppendorf tube. The biosensor cells mixed with the antibody are incubated at room temperature for 10 minutes and then loaded into the long loop portion of the reservoir card. For a positive control, 30 µL of anti-CD3ε antibody in RPMI media is loaded into the short loop portion of the reservoir card. The reservoir card is then locked into the test cartridge base. A 30 µL volume of the sample to be tested is added into the reaction chamber. The test cartridge base is inserted into the testing device and the biosensor cells are injected into the mixing chamber to initiate the reaction. The resulting signal is recorded for 4 to 8 minutes and at the end of the test period, the 30 µL of anti-CD3ε antibody is injected from the short loop of the reservoir into reaction chamber as a positive control reaction that is recorded for 2 minutes. As an alternative positive control, 30 µL of 0.61 mM Digitonin can be used rather than anti-CD3ε antibody. A negative control test can be performed using a predetermined pathogen that is not specific for the antibody being used.

### Example Bioassay 3: Coating Analyte with Antibodies

In another exemplary embodiment of the bioassay of the present invention, wherein the detector molecule is a soluble antibody, the analyte (e.g., pathogenic bacteria) is coated with the soluble antibody for a period of time prior to mixing the sample to be tested with the biosensor. In this embodiment, charged biosensor cells are centrifuged and concentrated to about 400,000 cells/180 µL (adequate for one reaction) in the charging medium. A 180 µL (about 400,000 cells) aliquot of the biosensor cells is loaded into the long loop portion of the reservoir card. For a positive control, 30 µL of anti-CD3ε antibody in RPMI media is loaded into the short loop portion of the reservoir card. The reservoir card is then locked into the cartridge base. A 2 µL volume of an antibody (at 0.5 mg/mL) against the target pathogen, such as anti-E. *coli* O111 (wherein the target pathogen is *E. coli* O111), is mixed with 28 µL of the sample to be tested in an Eppendorf tube. The sample is incubated at room temperature for 10 minutes then added into the cartridge mixing chamber. The cartridge is inserted into the PMT and the biosensor cells are injected into the mixing chamber to initiate the reaction. The resulting signal is recorded for 4 to 8 minutes and at the end of the test period, the 30 µL of anti-CD3ε antibody is injected from the short loop of the reservoir into reaction chamber as a positive control reaction that is recorded for 2 minutes. As an alternative positive control, 30 µL of 0.61 mM Digitonin can be used rather than anti-CD3ε antibody. A negative control test can be performed using a predetermined pathogen that is not specific for the antibody being used.

The exemplary bioassays described herein may include other additives that reduce background noise and enhance signal. Using anti-CD3ε antibody as a positive control, the system has been demonstrated to detect fewer than 10 charged biosensor cells in a mixture of 50,000 uncharged biosensor cells. The biosensor itself has been demonstrated to detect 230 CFU of bacteria in a sample of 30 µL. In the bioassays described above, a proprietary monoclonal antibody (1F11) against *E. coli* O111 bacteria was used to detect *E*. *coli* O111 bacteria with *E*. *coli* 0157 being used as a negative control. *E. coli* 0157 was demonstrated to give negative results, thereby proving the specificity of the system. Numerous commercially available antibodies may also be used with the described bioassay. With regard to the proprietary monoclonal antibody (1F11), antibody analysis and selection of the monoclonal antibody was accomplished as described below.

Antibody production was determined by an ELISA performed in 96-well multiwell plates. Each well was coated with different LPS (*E. coli* 0157, *E. coli* 0127, *E. coli* O111, *E. coli* O26, *Klebsiella pnuemoniae, Salmonella enterioa* and naive sera) or bacteria cells (*E. coli* 0157, *E. coli* O111, *E. coli* 26 and *E. coli* DH5α). Hybridoma supernatant from different clones of mAb 0157 or mAb O111 were added to the wells. Horseradish peroxidase-conjugated (HRP) goat anti-mouse IgG was used for detection (Appendix III.A.3). The two hybridoma clones (1B10 and 6G1) of *E. coli* 0157 exclusively recognize LPS of *E. coli* 0157 and *E. coli* 0157. The nine hybridoma clones of *E. coli* O111 specifically recognize LPS of *E. coli* O111 and *E. coli* O111. The clones from the highest optical density (OD) reading were chosen for validation, which was accomplished as described below.

The hybridoma cell pellets were collected and stored at -80°C before RNA extraction. The extracted RNA was used as a template for reverse transcription to cDNA, followed by nested PCR amplification. All positive PCR products were cloned into TA cloning vectors and sent for sequencing. The variable regions of the light chain and the heavy chain were determined after analysis of sequences. Four single chain antibodies (scFv) of 0157 (1B10) (customized mAb produced by FSC) and ATCC HB 10452, as well as two single chain antibodies of O111 produced by FSC (1F11 and 1F2) were recombinantly expressed and purified by Immobilized metal ion affinity chromatography (IMAC). The sequence of scFv was constructed as the following order: pel B secretion signal + amino acid Alanine + Histidine tag + amino acids Glycine-Serine-Serine-Glycine + TEV cleavage site + amino acids Glycine-Serine-Serine-Glycine + heavy chain variable region + Linker region Serine-Alanine-Aspartic Acid-Aspartic Acid-Alanine-lysine-lysine-Aspartic Acid-Alanine-Alanine-Lysine-Lysine-Aspartic Acid- Aspartic Acid -Alanine-Lysine-Lysine-Aspartic Acid- Aspartic Acid + light chain variable region. The purified scFvs were tested using multi-well plates coated with LPS of 0157 or O111.

The purpose of this study was to investigate the interactions of monoclonal antibody (mAb) to whole bacterial cells (*E. coli* 0157 or *E. coli* O111), and to estimate the kinetic constant (KD) of antibody-bacterial interaction. In these assays, goat anti-*E*. *coli* 0157 polyclonal antibody, goat anti-*E*. *coli* O111 polyclonal antibody, three monoclonal antibodies (1022, 1024 and 1061) against *E*. *coli* 0157, and one monoclonal antibody against *E*. *coli* O111 were used. A CM5 sensor chip and amine coupling kit were also used. All assays were performed on a Biacore X100 instrument. In the protocol, one polyclonal antibody (against a select bacteria) was immobilized onto a CM5 sensor chip. The select bacteria was then bound followed by injection of a monoclonal antibody against the same bacteria in a continuous buffer flow. The interaction was monitored in real time. The relative binding of the antibody to each bacterium was recorded in resonance units (RUs). Results of the BIAcore analysis of binding an *E. coli* 0157 specific antibody (mAb FF754) to *E. coli* 0157 and *E. coli* O111 indicated that 0157 mAb was specific for its target antigen.

### Fc Receptors

The receptors described herein for example, as ligands or detectors, can include alternative Fc-bearing chimeric receptors. The chimeric receptors described herein comprise an extracellular domain with binding affinity and specificity for the Fc portion of an immunoglobulin ("Fc binder"), a transmembrane domain, at least one co-stimulatory signaling domain, and a cytoplasmic signaling domain comprising an ITAM. The chimeric receptors are configured such that, when expressed on a host cell, the extracellular ligand-binding domain is located extracellularly for binding to a target molecule (e.g., an antibody or a Fc-fusion protein) and the co-stimulatory signaling domain and the ITAM-containing cytoplasmic signaling domain are located in the cytoplasm for triggering activation and/or effector signaling. In some embodiments, a chimeric receptor construct as described herein comprises, from N-terminus to C-terminus, the Fc binder, the transmembrane domain, the at least one co-stimulatory signaling domain, and the ITAM-containing cytoplasmic signaling domain. In other embodiments, a chimeric receptor construct as described herein comprises, from N-terminus to C-terminus, the Fc binder, the transmembrane domain, the ITAM-containing cytoplasmic signaling domains, and the at least one co-stimulatory signaling domain.

Any of the chimeric receptors described herein can further comprise a hinge domain, which can be located at the C-terminus of the Fc binder and the N-terminus of the transmembrane domain. Alternatively, or in addition, the chimeric receptor constructs described herein can contain two or more co-stimulatory signaling domains, which can link to each other or be separated by the ITAM-containing cytoplasmic signaling domain. The extracellular Fc binder, transmembrane domain, co-stimulatory signaling domain(s), and ITAM-containing cytoplasmic signaling domain in a chimeric receptor construct can be linked to each other directly, or via a peptide linker.

The chimeric receptor constructs described herein comprise an extracellular domain that is an Fc binder, i.e., capable of binding to the Fc portion of an immunoglobulin (e.g., IgG, IgA, IgM, or IgE) of a suitable mammal (e.g., human, mouse, rat, goat, sheep, or monkey). Suitable Fc binders can be derived from naturally occurring proteins such as mammalian Fc receptors or certain bacterial proteins (e.g., protein A, protein G). Additionally, Fc binders can be synthetic polypeptides engineered specifically to bind the Fc portion of any of the Ig molecules described herein with high affinity and specificity. For example, such an Fc binder can be an antibody or an antigen-binding fragment thereof that specifically binds the Fc portion of an immunoglobulin. Examples include, but are not limited to, a single-chain variable fragment (scFv), a domain antibody, or a nanobody. Alternatively, an Fc binder can be a synthetic peptide that specifically binds the Fc portion, such as a Kunitz domain, a small modular immunopharmaceutical (SMIP), an adnectin, an avimer, an affibody, a DARPin, or an anticalin, which can be identified by screening a peptide combinatory library for binding activities to Fc.

In some embodiments, the Fc binder is an extracellular ligand-binding domain of a mammalian Fc receptor. As used herein, an "Fc receptor" is a cell surface bound receptor that is expressed on the surface of many immune cells (including B cells, dendritic cells, natural killer (NK) cells, macrophage, neutorphils, mast cells, and eosinophils) and exhibits binding specificity to the Fc domain of an antibody. Fc receptors are typically comprised of at least 2 immunoglobulin (Ig)-like domains with binding specificity to an Fc (fragment crystallizable) portion of an antibody. In some instances, binding of an Fc receptor to an Fc portion of the antibody can trigger antibody dependent cell-mediated cytotoxicity (ADCC) effects. The Fc receptor used for constructing a chimeric receptor as described herein can be a naturally-occurring polymorphism variant (e.g., the CD16 V158 variant), which can have increased or decreased affinity to Fc as compared to a wild-type counterpart. Alternatively, the Fc receptor can be a functional variant of a wild-type counterpart, which carry one or more mutations (e.g., up to 10 amino acid residue substitutions) that alter the binding affinity to the Fc portion of an Ig molecule. In some instances, the mutation can alter the glycosylation pattern of the Fc receptor and thus the binding affinity to Fc.

The table below lists a number of exemplary polymorphisms in Fc receptor extracellular domains (see, e.g., Kim etal., J. Mol. Evol. 53:1-9, 2001):

**TABLE 1**

| Exemplary Polvmorohisms in Fc Receptors | | | | | | | | | | |
|---|---|---|---|---|---|---|---|---|---|---|
| Amino Acid Number | 19 | 48 | 65 | 89 | 105 | 130 | 134 | 141 | 142 | 158 |
| FCR10 | R | S | D | I | D | G | F | Y | T | V |
| P08637 | R | S | D | I | D | G | F | Y | I | F |
| S76824 | R | S | D | I | D | G | F | Y | I | V |
| J04162 | R | N | D | V | D | D | F | H | I | V |
| M31936 | S | S | N | I | D | D | F | H | I | V |
| M24854 | S | S | N | I | E | D | S | H | I | V |
| X07934 | R | S | N | I | D | D | F | H | I | V |
| X14356 (FcγRII) | N | N | N | S | E | S | S | S | I | I |
| M31932 (FcγRI) | S | T | N | R | E | A | F | T | I | G |
| X06948 (Fcα∈I) | R | S | E | S | Q | S | E | S | I | V |

Fc receptors are classified based on the isotype of the antibody to which it is able to bind. For example, Fc-gamma receptors (FcyR) generally bind to IgG antibodies, such as one or more subtype thereof (i.e., IgG1, IgG2, IgG3, IgG4); Fc-alpha receptors (FcαR) generally bind to IgA antibodies; and Fc-epsilon receptors (FccR) generally bind to IgE antibodies. In some embodiments, the Fc receptor is an Fc-gamma receptor, an Fc-alpha receptor, or an Fc-epsilon receptor. Examples of Fc-gamma receptors include, without limitation, CD64A, CD64B, CD64C, CD32A, CD32B, CD16A, and CD16B. An example of an Fc-alpha receptor is FcαR1/CD89. Examples of Fc-epsilon receptors include, without limitation, FcεRI and Fc.epsilon.RII/CD23. The table below lists exemplary Fc receptors for use in constructing the chimeric receptors described herein and their binding activity to corresponding Fc domains:

**TABLE 2**

| | Exemplary Fc Receptors | |
|---|---|---|
| Receptor name | Principal antibody ligand | Affinity for ligand |
| FcγRI (CD64) | IgG1 and IgG3 | High (Kd∼10⁻⁹ M) |
| FcγRIIA (CD32) | IgG | Low (Kd > 10⁻⁷ M) |
| FcγRIIB1 (CD32) | IgG | Low (Kd > 10⁻⁷ M) |
| FcγRIIB2 (CD32) | IgG | Low (Kd > 10⁻⁷ M) |
| FeγRIIIA (CD16a) | IgG | Low (Kd > 10⁻⁶ M) |
| FeyRIIIB (CD16b) | IgG | Low (Kd > 10⁻⁶ M) |
| FcεRI | IgE | High (Kd~10⁻¹⁰ M) |
| FcεRII (CD23) | IgE | Low (Kd > 10⁻⁷ M) |
| FcαRI (CD89) | IgA | Low (Kd > 10⁻⁶ M) |
| Fcα/µR | IgA and IgM | High for IgM, Mid for IgA |
| FeRn | IgG | |

Selection of the ligand binding domain of an Fc receptor for use in the chimeric receptors described herein will be apparent to one of skill in the art. For example, it can depend on factors such as the isotype of the antibody to which binding of the Fc receptor is desired and the desired affinity of the binding interaction. In some examples, (a) is the extracellular ligand-binding domain of CD16 incorporating a naturally occurring polymorphism that can modulate affinity for Fc. In some examples, (a) is the extracellular ligand-binding domain of CD 16 incorporating a polymorphism at position 158 (e.g., valine or phenylalanine). In some embodiments, (a) is produced under conditions that alter its glycosylation state and its affinity for Fc. In some embodiments, (a) is the extracellular ligand-binding domain of CD 16 incorporating modifications that render the chimeric receptor incorporating it specific for a subset of IgG antibodies. For example, mutations that increase or decrease the affinity for an IgG subtype (e.g., IgG1) can be incorporated.

In other embodiments, the Fc binder is derived from a naturally occurring bacterial protein that is capable of binding to the Fc portion of an IgG molecule. A Fc binder for use in constructing a chimeric receptor as described herein can be a full-length protein or a functional fragment thereof. Protein A is a 42 kDa surface protein originally found in the cell wall of the bacterium Staphylococcus aureus. It is composed of five domains that each fold into a three-helix bundle and are able to bind IgG through interactions with the Fc region of most antibodies as well as the Fab region of human VH3 family antibodies. Protein G is an approximately 60-kDa protein expressed in group C and G Streptococcal bacteria that binds to both the Fab and Fc region of mammalian IgGs. While native protein G also binds albumin, recombinant variants have been engineered that eliminate albumin binding.

Fc binders for use in chimeric receptors can also be created de novo using combinatorial biology or directed evolution methods. Starting with a protein scaffold (e.g., an scFv derived from IgG, a Kunitz domain derived from a Kunitz-type protease inhibitor, an ankyrin repeat, the Z domain from protein A, a lipocalin, a fibronectin type III domain, an SH3 domain from Fyn, or others), amino acid side chains for a set of residues on the surface can be randomly substituted in order to create a large library of variant scaffolds. From large libraries it is possible to isolate rare variants with affinity for a target like the Fc domain by first selecting for binding, followed by amplification by phage, ribosome or cell display. Repeated rounds of selection and amplification can be used to isolate those proteins with the highest affinity for the target.

Any of the Fc binders described herein can have a suitable binding affinity for the Fc portion of a therapeutic antibody. As used herein, "binding affinity" refers to the apparent association constant or K_{A}. The K_{A} is the reciprocal of the dissociation constant, K_{D}. The extracellular ligand-binding domain of an Fc receptor domain of the chimeric receptors described herein can have a binding affinity K_{D} of at least 10⁻⁵, 10⁻⁶, 10⁻⁷, 10⁻⁸, 10⁻⁹, 10^{-10M} or lower for the Fc portion of antibody. In some embodiments, the Fc binder has a high binding affinity for antibody, isotype of antibodies, or subtype(s) thereof, as compared to the binding affinity of the Fc binder to another antibody, isotype of antibodies or subtypes thereof. In some embodiments, the extracellular ligand-binding domain of an Fc receptor has specificity for an antibody, isotype of antibodies, or subtype(s) thereof, as compared to binding of the extracellular ligand-binding domain of an Fc receptor to another antibody, isotype of antibodies, or subtypes thereof. Fc-gamma receptors with high affinity binding include CD64A, CD64B, and CD64C. Fc-gamma receptors with low affinity binding include CD32A, CD32B, CD16A, and CD16B. An Fc-epsilon receptor with high affinity binding is Fc.epsilon.RI, and an Fc-epsilon receptor with low affinity binding is Fc.epsilon.RII/CD23.

The binding affinity or binding specificity for an Fc receptor or a chimeric receptor comprising an Fc binder (e.g., an extracellular ligand-binding domain of an Fc receptor) can be determined by a variety of methods including equilibrium dialysis, equilibrium binding, gel filtration, ELISA, surface plasmon resonance, or spectroscopy.

In some embodiments, the extracellular ligand-binding domain of an Fc receptor comprises an amino acid sequence that is at least 90% (e.g., 91, 92, 93, 94, 95, 96, 97, 98, 99%) identical to the amino acid sequence of the extracellular ligand-binding domain of a naturally-occurring Fc-gamma receptor, an Fc-alpha receptor, or an Fc-epsilon receptor. The "percent identity" of two amino acid sequences can be determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of the disclosure. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

Still another embodiment provides a system that includes a space or compartment for contacting an engineered cell with an analyte; an engineered cell that comprises a ligand, a signal transduction pathway, and a reporter; wherein the universal detector element binds to a predetermined analyte, the signal-transduction pathway receives a first signal induced by binding of the analyte to the ligand, transmits the first signal to the reporter, and the reporter emits a second detectable signal upon receipt of the first signal from the signal transduction pathway; and a detector.

Still another embodiment provides a system that includes a space or compartment for contacting an engineered cell with a pre-determined analyte; an engineered cell that comprises an aggregation of ligands and signal transduction elements that constitutively transmit a signal to a detector that emits light or another detectable signal; and a detector; wherein binding of the pre-determined analyte to the aggregation of ligands and signal transduction elements attenuates signal transduction and attenuates the emission of light or other detectable signal by the reporter. The aggregation of ligands and signal transduction elements is maintained by a cohesive adaptor and when the cohesive adaptor is bound by the predetermined analyte, its ability to maintain the aggregation of ligands and signal transduction elements is attenuated.

Still another embodiment provides a system that includes a space or compartment for contacting an engineered cell with a pre-determined analyte; an engineered cell that comprises a ligand, a signal transduction element that transmits an inhibitory signal when bound to the pre-determined analyte, and a reporter that constitutively emits light or another detectable signal; a detector; wherein binding of the pre-determined analyte to the universal detector elementinduces an inhibitory signal that attenuates the emission of light or other detectable signal by the reporter. In this embodiment, the universal detector element can include an immunoreceptor tyrosine-based inhibition motif (ITIM). ITIMs are further described in Staub E, Rosenthal A, Hinzmann B (2004)."Systematic identification of immunoreceptor tyrosine-based inhibitory motifs in the human proteome". Cell Signal 16 (4): 435-456.

## Claims

1. A biosensor system for the detection of target analytes, comprising:
(a) a living biological cell of a predetermined type;
(b) a signal-generating reporter within the living biological cell, wherein the signal-generating reporter is responsive to predetermined changes occurring within the living biological cell;
(c) a signal transduction pathway or activator mechanism associated with the signal-generating reporter, wherein the signal transduction pathway or activator mechanism is operative to induce the predetermined changes within the living biological cell;
(d) a universal detector element associated with the activator mechanism, wherein the universal detector element is operative to trigger the activator mechanism, and wherein the universal detector element comprises an antibody variable diversity joining (VDJ) region or Fab fragment;
(e) an analyte binding element associated with the universal detector element, wherein the analyte binding element is specific to both the universal detector element and a target analyte; and
(f) wherein upon the binding of an analyte binding element to which a target analyte is also bound to the universal detector element, the universal detector element triggers the activator mechanism causing the predetermined changes to occur within the living biological cell, thereby causing the signal-generating reporter to generate a detectable signal,
wherein the cell is not the human body at the various stages of its formation and development.

2. The biosensor system of claim 1, wherein the living biological cell is a prokaryotic cell; a eukaryotic cell; a yeast cell; an insect cell; a mammalian cell; an animal cell; a plant cell; a non-reproducing cell; a fixed cell; a drug-treated cell; a chemically-treated cell; an osmotically-treated cell; a radiated cell; an artificial cell; a synthetic cell; a follicular dendritic cell; a natural killer cell; a macrophage; a monocyte; a mononuclear phagocyte; a neutrophil; an eosinophil; or a basophil, optionally wherein (a) the insect cell is a Drosophila Schneider 2 cell or an sf9 cell, or (b) the mammalian cell is an HEK cell; a CHO cell; a COS cell; a 3T3 cell, or other cultured, immortalized, or passaged mammalian cell.

3. The biosensor system of claim 1, wherein the signal-generating reporter is a dye having fluorescent, ultraviolet, or visible properties; an enzyme adapted to produce a luminescent or fluorescent signal; fluorescent, charged or magnetic nanoparticles, nanodots, or quantum dots; a fluorescent protein; or other calcium-sensitive luminescent or fluorescent molecule, optionally wherein (a) the fluorescent protein is green fluorescent protein, or (b) the signal-generating reporter is obelin; thalassicolin; mitrocomin (halistaurin); clytin (phialidin); mnemopsin; berovin; Indo-1; Fura-2; Quin-2; Fluo-3; Rhod-2; calcium green; BAPTA; cameleons, or other calcium-sensitive luminescent or fluorescent molecule.

4. The biosensor system of claim 1, wherein the signal transduction pathway or activator mechanism is activated by at least one of: a change in pH or temperature of the living biological cell; a change in electrical or magnetic properties of the living biological cell; activation of a G-protein-coupled receptor signaling pathway within the living biological cell; activation of a phosphatidyl inositol pathway within the living biological cell; activation of a signal transduction pathway within the living biological cell that releases diacylglycerol, ceramide, or other lipophilic messenger molecule; activation of a signal transduction pathway within the living biological cell that releases or produces nitric oxide, cAMP, cGMP, or other cyclic nucleotide; activation of a signal transduction pathway within the living biological cell that releases or produces superoxide, hydrogen peroxide, carbon monoxide, hydrogen sulfide, or other secondary redox signaling molecule; or a conformational change in a receptor expressed by the living biological cell, wherein the conformational change occurs only after the universal detector element has bound to a target analyte.

5. The biosensor system of claim 1, wherein the universal detector element comprises at least one of: a synthetic peptide; a non-peptide organic determinant of a known size; a lectin determinant, a carbohydrate-binding module, or other carbohydrate-binding determinant; a lipid-binding determinant; or a metallothione determinant that binds a metal or other metal-binding determinant.

6. The biosensor system of claim 1, wherein the analyte binding element is an affibody; an aptamer; or a soluble receptor, or wherein the analyte binding element includes an IgG fragment that is a single chain antibody or a single chain diabody.

7. The biosensor system of claim 1, wherein the target analyte is a beneficial gut bacterium; a pathogenic bacterium; a protein biomarker; a small molecule toxin, metabolite, or chemical warfare agent; or a small molecule linked to a macromolecular carrier.

## Patentansprüche

1. Biosensorsystem zum Nachweisen von Zielanalyten, umfassend:
(a) eine lebende biologische Zelle eines vorbestimmten Typen;
(b) einen signalerzeugenden Reporter innerhalb der lebenden biologischen Zelle, wobei der signalerzeugende Reporter auf vorbestimmte Änderungen reagiert, die innerhalb der biologischen Zelle auftreten;
(c) einen Signaltransduktionsweg oder Aktivatormechanismus, verknüpft mit dem signalerzeugenden Reporter, wobei der Signaltransduktionsweg oder Aktivatormechanismus dazu funktionsfähig ist, die vorbestimmten Änderungen innerhalb der lebenden biologischen Zelle zu induzieren;
(d) ein universelles Detektorelement, verknüpft mit dem Aktivatormechanismus, wobei das universelle Detektorelement dazu funktionsfähig ist, den Aktivatormechanismus auszulösen, und wobei das universelle Detektorelement eine Antikörper-VDJ(variable diversity joining)-Region oder ein Fab-Fragment umfasst;
(e) ein Analytbindungselement, verknüpft mit dem universellen Detektorelement, wobei das Analytbindungselement sowohl für das universelle Detektorelement als auch für einen Zielanalyten spezifisch ist; und
(f) wobei bei der Bindung eines Analytbindungselements, an das auch ein Zielanalyt gebunden ist, an das universelle Detektorelement das universelle Detektorelement den Aktivatormechanismus auslöst, wodurch die vorbestimmten Änderungen innerhalb der lebenden biologischen Zelle auftreten und der signalerzeugende Reporter ein nachweisbares Signal erzeugt,
wobei die Zelle nicht der menschliche Körper in den verschiedenen Stadien seiner Entstehung und Entwicklung ist.

2. Biosensorsystem nach Anspruch 1, wobei die lebende biologischen Zelle eine prokaryotische Zelle; ein eukaryotische Zelle; eine Hefezelle; eine Insektenzelle; eine Säugerzelle; eine tierische Zelle; eine pflanzliche Zelle; eine nichtreproduzierende Zelle; eine fixierte Zelle; eine mit Arzneimitteln behandelte Zelle; eine chemisch behandelte Zelle; eine osmotisch behandelte Zelle; eine bestrahlte Zelle; eine künstliche Zelle; eine synthetische Zelle; eine follikuläre dendritische Zelle; eine natürliche Killerzelle; ein Makrophage; ein Monozyt; ein mononukleärer Phagozyt; eine neutrophile Zelle; eine eosinophile Zelle; oder eine basophile Zelle ist, optional wobei (a) die Insektenzelle eine Drosophila-Schneider-2-Zelle oder eine sf9-Zelle ist, oder
(b) die Säugerzelle eine HEK-Zelle, eine CHO-Zelle, eine COS-Zelle, eine 3T3-Zelle oder eine andere kultivierte, immortalisierte oder passagierte Säugerzelle ist.

3. Biosensorsystem nach Anspruch 1, wobei der signalerzeugende Reporter ein Farbstoff mit fluoreszenten, ultravioletten oder sichtbaren Eigenschaften; ein Enzym, angepasst zum Erzeigen eines lumineszenten oder fluoreszenten Signals; fluoreszente, geladene oder magnetische Nanopartikel, Nanodots oder Quantendots; ein fluoreszentes Protein oder ein anderes calciumempfindliches oder lumineszentes oder fluoreszentes Molekül ist, optional wobei (a) das fluoreszente Protein ein grünes fluoreszentes Protein ist; oder (b) der signalerzeugende Reporter Obelin; Thalassicolin; Mitrocomin (Halistaurin); Clytin (Phialidin); Mnemopsin; Berovin; Indo-1; Fura-2; Quin-2; Fluo-3; Rhod-2; Calciumgrün; BAPTA; Cameleone oder ein anderes calciumempfindliches oder lumineszentes oder fluoreszentes Molekül ist.

4. Biosensorsystem nach Anspruch 1, wobei der Signaltransduktionsweg oder Aktivatormechanismus aktiviert wird durch mindestens eines von: einer Änderung des pH-Werts oder der Temperatur der lebenden biologischen Zelle; einer Änderung der elektrischen oder der magnetischen Eigenschaften der lebenden biologischen Zelle; Aktivierung eines G-Protein-gekoppelten Rezeptorsignalisierungswegs innerhalb der lebenden biologischen Zelle; Aktivierung eines Phosphatidylinositolwegs innerhalb der lebenden biologischen Zelle; Aktivierung eines Signaltransduktionswegs innerhalb der lebenden biologischen Zelle, der Diacylglycerol, Ceramid oder andere lipophile Botenmoleküle freisetzt; Aktivierung eines Signaltransduktionswegs innerhalb der lebenden biologischen Zelle, der Stickstoffoxid, cAMP, cGMP oder andere zyklische Nukleotide freisetzt oder erzeugt; Aktivierung eines Signaltransduktionswegs innerhalb der lebenden biologischen Zelle, der Superoxid, Wasserstoffperoxid, Kohlenmonoxid, Schwefelwasserstoff oder andere sekundäre Redoxsignalisierungsmoleküle freisetzt oder erzeugt; oder einer Konformationsänderung in einem Rezeptor, der von der lebenden biologischen Zelle exprimiert wird, wobei die Konformationsänderung erst nach dem Binden des universellen Detektorelements an einen Zielanalyten auftritt.

5. Biosensorsystem nach Anspruch 1, wobei das universelle Detektorelement mindestens eines umfasst von: einem synthetischen Peptid; einer nichtpeptidischen organischen Determinante bekannter Größe; einer Lektin-Determinante, einem kohlenhydratbindenden Modul oder einer anderen kohlenhydratbindenden Determinante; einer lipidbindenden Determinante; oder einer Metallothiondeterminante, die ein Metall oder eine andere metallbindende Determinante bindet.

6. Biosensorsystem nach Anspruch 1, wobei das Analytbindungselement ein Affibody; ein Aptamer; oder ein löslicher Rezeptor ist, oder wobei das Analytbindungselement ein IgG-Fragment ist, das einen Einzelketten-Antikörper oder einen Einzelketten-Diabody einschließt.

7. Biosensorsystem nach Anspruch 1, wobei der Zielanalyt ein nützliches Darmbakterium; ein pathogenes Bakterium; ein Proteinbiomarker; ein niedermolekulares Toxin; ein Metabolit oder ein chemischer Kampfstoff; oder eine an einen makromolekularen Träger gebundene niedermolekulare Verbindung ist.

## Revendications

1. Système de biocapteur pour la détection d'analytes cibles, comprenant :
(a) une cellule biologique vivante d'un type prédéterminé ;
(b) un rapporteur générateur de signal dans la cellule biologique vivante, le rapporteur générateur de signal étant sensible à des changements prédéterminés ayant lieu dans la cellule biologique vivante ;
(c) une voie ou un mécanisme activateur de transduction de signal associé(e) au rapporteur générateur de signal, la voie ou le mécanisme activateur de transduction de signal fonctionnant pour induire les changements prédéterminés dans la cellule biologique vivante ;
(d) un élément déflecteur universel associé au mécanisme activateur, l'élément détecteur universel fonctionnant pour déclencher le mécanisme activateur, et l'élément détecteur universel comprenant une région de jonction de diversité variable (VDJ) d'anticorps ou un fragment Fab ;
(e) un élément de liaison à un analyte associé à l'élément détecteur universel, l'élément de liaison à un analyte étant spécifique à la fois à l'élément détecteur universel et à un analyte cible ; et
(f) lors de la liaison d'un élément de liaison à un analyte auquel un analyte cible est également lié à l'élément détecteur universel, l'élément détecteur universel déclenche le mécanisme activateur ramenant les changements prédéterminés à avoir lieu dans la cellule biologique vivante, ramenant ainsi le rapporteur de génération de signal à générer un signal détectable,
la cellule n'étant pas le corps humain à divers stades de sa formation et de son développement.

2. Système de biocapteur selon la revendication 1, la cellule biologique vivante étant une cellule procaryote ; une cellule eucaryote ; une cellule de levure ; une cellule d'insecte ; une cellule de mammifère ; une cellule animale ; une cellule végétale ; une cellule non reproductrice ; une cellule fixe ; une cellule traitée par un médicament ; une cellule traitée chimiquement ; une cellule traitée osmotiquement ; une cellule rayonnée ; une cellule artificielle ; une cellule synthétique ; une cellule dendritique folliculaire ; une cellule tueuse naturelle ; un macrophage ; un monocyte ; un phagocyte mononucléaire ; un neutrophile ; un éosinophile ; ou un basophile, éventuellement (a) la cellule d'insecte étant une cellule de drosophile Schneider 2 ou une cellule sf9, ou (b) la cellule de mammifère étant une cellule HEK ; une cellule CHO ; une cellule COS ; une cellule 3T3, ou une autre cellule de mammifère cultivée, immortalisée ou passagère.

3. Système de biocapteur selon la revendication 1, le rapport de génération de signal étant un colorant ayant des propriétés fluorescentes, ultraviolettes, ou visibles ; une enzyme adaptée pour produire un signal luminescent ou fluorescent ; des nanoparticules, des nanopoints ou des points quantiques fluorescents, chargés ou magnétiques ; une protéine fluorescente ; ou une autre molécule luminescente ou fluorescente sensible au calcium, éventuellement (a) la protéine fluorescente étant une protéine fluorescente verte, ou (b) le rapporteur générateur de signal étant l'obéline ; la thalassicoline ; la mitrocomine (halistaurine) ; la clytine (phialidine) ; la mnémopsine ; la bérovine ; Indo-1 ; Fura-2 ; Quin-2 ; Fluo-3 ; Rhod-2 ; le vert de calcium ; BAPTA ; des caméléons, ou une autre molécule luminescente ou fluorescente sensible au calcium.

4. Système de biocapteur selon la revendication 1, la voie ou le mécanisme activateur de transduction de signal étant activé(e) par au moins l'un parmi : un changement du pH ou de la température de la cellule biologique vivante ; un changement des propriétés électriques ou magnétiques de la cellule biologique vivante ; l'activation d'une voie de signalisation des récepteurs couplés aux protéines G dans la cellule biologique vivante ; l'activation d'une voie de phosphatidylinositol dans la cellule biologique vivante ; l'activation d'une voie de transduction de signal dans la cellule biologique vivante qui libère du diacylglycérol, du céramide ou d'autres molécules messagères lipophiles ; l'activation d'une voie de transduction de signal dans la cellule biologique vivante qui libère ou produit de l'oxyde nitrique, de l'AMPc, de la GMPc ou d'autres nucléotides cycliques ; l'activation d'une voie de transduction de signal dans la cellule biologique vivante qui libère ou produit du superoxyde, du peroxyde d'hydrogène, du monoxyde de carbone, du sulfure d'hydrogène ou une autre molécule secondaire de signalisation redox ; ou un changement conformationnel d'un récepteur exprimé par la cellule biologique vivante, le changement conformationnel ayant lieu seulement après que l'élément détecteur universel s'est lié à un analyte cible.

5. Système de biocapteur selon la revendication 1, l'élément détecteur universel comprenant au moins l'un parmi : un peptide synthétique ; un déterminant organique non peptidique d'une taille connue ; un déterminant de lectine, un module de liaison aux glucides ou un autre déterminant de liaison aux glucides ; un déterminant de liaison aux lipides ; ou un déterminant de métallothione qui lie un métal ou un autre déterminant de liaison à un métal.

6. Système de biocapteur selon la revendication 1, l'élément de liaison à un analyte étant un affibody, un aptamère ou un récepteur soluble, ou l'élément de liaison à un analyte comportant un fragment d'IgG qui est un anticorps monocaténaire ou un dianticorps monocaténaire.

7. Système de biocapteur selon la revendication 1, l'analyte cible étant une bactérie intestinale bénéfique ; une bactérie pathogène ; un biomarqueur protéique ; une toxine à petite molécule, un métabolite ou un agent de guerre chimique ; ou une petite molécule liée à un support macromoléculaire.
